# EUROPEAN PATENT APPLICATION

(11) **EP 3 854 407 A1**
(43) Date of publication of application: **28.07.2021**
(21) Application number: 20305061.2
(22) Date of filing: 24.01.2020
(51) Int. Cl.: A61K 35/74, A61K 35/741, C07K 14/195

(54) **MICROBIOTA-DERIVED PROTEINS INDUCING IL-10 RELEASE FROM HUMAN CELLS AND USES THEREOF**

(71) Applicant: Enterome S.A., 75011 Paris (FR)
(72) Inventor: CULTRONE, Antonietta, 94240 L'Hay Les Roses (FR); CHENE, Laurent, 45170 Neuville aux Bois (FR); STROZZI, Francesco, 75003 Paris (FR); BONNY, Christophe, 75011 Paris (FR)
(74) Representative: Office Freylinger

(57) **Abstract**

The present invention relates to microbiota-derived proteins, which are capable of inducing and/or enhancing secretion of IL-10 from human cells. Accordingly, the present invention provides microbial proteins, and fragments and sequence variants thereof, which stimulate IL-10 release from human immune cells. The present invention also relates to nucleic acids encoding such proteins, cells expressing such proteins, respective pharmaceutical compositions and uses thereof.

SEQUENCE LISTING

<110>  ENTEROME SA

<120>  MICROBIOTA-DERIVED PROTEINS INDUCING IL-10 RELEASE FROM HUMAN
       CELLS AND USES THEREOF

<130>  P-GRAF-007/EP

<160>  20

<170>  PatentIn version 3.5

<210>  1
<211>  121
<212>  PRT
<213>  microbiota

<400>  1

Ala Phe Leu Phe Thr Ser Thr Gly Val Pro Lys Lys Ala Ala Glu Ala
1               5                   10                  15

Ala Phe Phe Leu Tyr Leu Asn Lys Gly Thr Lys Lys Gly Ser Arg Ser
            20                  25                  30

Cys Phe Phe Ile Tyr Leu Asp Arg Gly Thr Lys Lys Gly Ser Arg Ser
        35                  40                  45

Cys Phe Phe Tyr Leu Pro Arg Gln Gly Tyr Gln Lys Gly Ser Arg Gly
    50                  55                  60

Cys Phe Phe Ile Tyr Leu Asp Arg Gly Thr Lys Lys Gly Ser Arg Gly
65                  70                  75                  80

Cys Phe Phe Ile Tyr Leu Asp Cys Glu Lys Arg Ala Gly Asn Val Cys
                85                  90                  95

Ile Arg Lys Cys Arg Gly Arg Tyr Leu His Lys Lys Thr Pro Arg Arg
            100                 105                 110

Tyr Arg Asn Ala Glu Ala Thr Cys Ser
        115                 120

<210>  2
<211>  100
<212>  PRT
<213>  microbiota

<400>  2

Gln Thr Arg Lys Gln Arg Glu Asp Ala Lys Arg Glu Ala Trp Lys Lys
1               5                   10                  15

Glu Arg Lys Glu Lys Lys Ala Leu Glu Ala Gln Gln Asp Ser Val Ser
            20                  25                  30

Phe Met Lys Asp Thr Glu Ser Cys Cys Ala Ser Lys Ala Phe Phe Ser
        35                  40                  45

Leu Arg Ser Phe Phe His Ala Ser Arg Leu Ala Ser Ser Leu Cys Phe
    50                  55                  60

Leu Val Cys Ala Phe Val Thr Pro Leu Lys Gln Thr Ser Asn Asn Ala
65                  70                  75                  80

Ala Lys Asn Asn Thr Phe Phe Ile Ile Lys Ala Val Leu Leu Ile Asn
                85                  90                  95

Ile Ser Phe Arg
            100

<210> 3
<211> 78
<212> PRT
<213> microbiota

<400> 3

Ala Arg Asn Tyr Thr Cys Asp Val Cys Gly Asn Gly Thr Ile Gln Ile
1               5                   10                  15

Val Ser Ser His Ile Ile His Asn Val His Cys Gly Phe Ile Pro Cys
            20                  25                  30

Asn Lys Ile Asn Gly Val Met Asp Glu Val Val Tyr Lys Thr Val Thr
        35                  40                  45

Glu Asn Asn Glu Ala Cys Asn Asn Cys Gly Val Ser Tyr Thr Tyr Lys
    50                  55                  60

Val Tyr Gly Asp Met Glu Ile Ile Cys Lys Ala Lys Ala Asn
65                  70                  75

<210> 4
<211> 84
<212> PRT
<213> microbiota

<400> 4

Ala Glu Pro Ala Asp Thr Ala Ile Ser Glu Arg Arg Val Glu Leu Cys
1               5                   10                  15

Gly Asn Cys Gly Gly Arg Met Val Thr Ser Thr Thr Trp Gly Ser Trp

            20                  25                  30

Tyr Thr Val Ala Gln Ile Lys Cys Thr His His Asn Tyr Gly Thr Asp
        35                  40                  45

Leu Arg Gln Gln Arg Asp Gly Thr Ala Thr Thr Lys Cys Gln Gly Cys
    50                  55                  60

Gly Gln Gly Tyr Thr Thr Ser Lys Ser Gln Thr Arg Ile Val Cys His
65                  70                  75                  80

Gly Tyr Asp Ser

<210> 5
<211> 144
<212> PRT
<213> microbiota

<400> 5

Ala Ala Phe Val Phe Ser Asn Ser Leu Lys Pro Ala Asn Ala Ser Ser
1               5                   10                  15

Ala Glu Ser Ser Arg Leu Leu Ile His Val Asn Ser Phe Phe Ser Gln
            20                  25                  30

Leu Gly Leu Lys Pro Ile Ser Glu Asn Leu Leu Arg Lys Thr Ala His
        35                  40                  45

Phe Cys Glu Phe Gly Met Leu Gly Ile Leu Ala Ser Ser Ala Cys Ala
    50                  55                  60

Met Phe Ser Gly Ala Tyr Ser Ala Ala Ser Leu Pro Ser Leu Arg Arg
65                  70                  75                  80

Arg Gly Phe Phe Ile Ser Phe Gly Val Ser Val Ala Cys Ala Val Cys
                85                  90                  95

Asp Glu Thr Ile Gln Tyr Phe Val Pro Gly Arg Ala Cys Arg Val Thr
            100                 105                 110

Asp Met Leu Ile Asp Ser Ala Gly Ala Leu Cys Gly Leu Ala Ala Val
        115                 120                 125

Leu Ala Phe Cys Ala Ala Ile Arg Val Arg Arg Arg Arg Arg Arg Asn
    130                 135                 140

<210> 6

<211>  58
<212>  PRT
<213>  microbiota

<400>  6

Leu Ala Gly Pro Gly Ser Gly Cys Arg Phe Thr Pro Ser Cys Ser Thr
1               5                   10                  15

Tyr Phe Ile Gln Ala Val Glu Ile His Gly Ala Leu Lys Gly Ser Leu
            20                  25                  30

Met Gly Ile Trp Arg Ile Leu Arg Cys Asn Pro Trp Gly Gly Cys Gly
        35                  40                  45

Tyr Asp Pro Val Pro Pro Arg Lys Pro Arg
    50                  55

<210>  7
<211>  44
<212>  PRT
<213>  microbiota

<400>  7

Ala Lys Leu Gly Met Ala Ala Gly Ala Met Leu Val Leu Gly Leu Leu
1               5                   10                  15

Ala Ala Gly Ala Ser Gly Gly Thr Leu Ile Leu Ala Ala Leu Ala Leu
            20                  25                  30

Cys Ala Val Thr Leu Leu Cys Gly Arg Lys Lys Gln
        35                  40

<210>  8
<211>  75
<212>  PRT
<213>  microbiota

<400>  8

Val Glu Lys Lys Thr Val Ile Thr Lys Cys Ala Ile Thr Val Asn Glu
1               5                   10                  15

Tyr Arg Glu Lys Val Val Pro Ser Met Arg Lys Ile His Ala Ile Val
            20                  25                  30

Ile Phe Val Ser Tyr Ser Ile Asn His Leu Tyr Lys Asn Cys Glu Pro
        35                  40                  45

Glu Gln Leu Phe Ser Pro Gly Arg Lys Thr Lys Lys Pro Pro Pro Ala
    50                  55                  60

Thr Cys Arg Lys Arg Leu Asn Leu Gln Tyr Phe
65                  70                  75

<210>  9
<211>  96
<212>  PRT
<213>  microbiota

<400>  9

Glu Ile Thr Gln Pro Cys Asn His Val Lys Ser Asp Trp Ile Ile Asp
1               5                   10                  15

Lys Glu Ala Thr Cys Ile Gly Ser Tyr Ala Phe Tyr Asn Cys Thr Ser
            20                  25                  30

Leu Thr Ser Ile Glu Ile Ser Thr Ser Val Thr Lys Ile Lys Tyr Arg
        35                  40                  45

Ala Phe Ala Ser Cys Arg Ala Leu Asn Asn Ile Tyr Tyr Thr Gly Thr
    50                  55                  60

Leu Thr Gln Trp Asn Glu Ile Ser Lys Asp Thr Asn Trp Asn Trp Ala
65                  70                  75                  80

Ala Pro Leu Asn Cys Lys Val Ile Cys Leu Asn Gly Thr Cys Tyr Leu
                85                  90                  95

<210>  10
<211>  98
<212>  PRT
<213>  microbiota

<400>  10

Leu Leu Val Ser Val Cys Thr Ala Ala Gly Leu Leu Ala Val Ala Met
1               5                   10                  15

Arg Gln Ile Glu Pro Leu Leu Ala Trp Leu Arg Thr Leu Glu Val Tyr
            20                  25                  30

Phe Gln Gly Gln Ser Pro Ala Val Leu Leu Arg Ala Leu Gly Ile Ala
        35                  40                  45

Leu Val Ala Gln Phe Ala Ala Asp Thr Cys Arg Glu Ala Gly Leu Cys
    50                  55                  60

Ala Ala Ser Thr Ala Ile Glu Leu Cys Gly Arg Val Leu Val Leu Leu
65                  70                  75                  80

Gln Ala Leu Pro Leu Leu Arg Ser Leu Leu Gly Ser Phe Ala Asp Tyr
                85                  90                  95

Leu Gln

<210>  11
<211>  366
<212>  DNA
<213>  microbiota

<400>  11
gcttttttat ttacctcgac aggggtacca aaaaaggcag cagaggctgc tttttttctt      60

tacctcaaca agggtaccaa aaaaggcagc agaagctgct tttttattta cctcgacagg      120

ggtaccaaaa aaggcagcag aagctgcttt ttttatttac ctcgacaggg gtaccaaaaa      180

ggcagcagag gctgcttttt tatttacctc gacaggggta ccaaaaaagg tagcagaggc      240

tgctttttta tttacctcga ttgcgaaaag cgcgcaggca atgtctgcat acgcaaatgc      300

cgcgggcggt atttgcacaa aaaaacgccg cggcggtatc gaaacgccga ggcgacgtgc      360

tcctaa                                                                366

<210>  12
<211>  303
<212>  DNA
<213>  microbiota

<400>  12
caaaccagaa aacaaagaga agatgccaaa cgcgaagcat ggaaaaaaga acgtaaggaa      60

aagaaggcct tggaagcaca gcaagattcc gtgtctttca tgaaagacac ggaatcttgc      120

tgtgcttcca aggccttctt ttccttacgt tcttttttcc atgcttcgcg tttggcatct      180

tctctttgtt ttctggtttg tgcatttgtt actccgctga agcaaacaag taacaatgct      240

gctaagaata atactttttt cataattaaa gctgttttgt taatcaatat atcttttaga      300

taa                                                                   303

<210>  13
<211>  237
<212>  DNA
<213>  microbiota

<400>  13
gcaaggaact atacatgtga tgtatgtgga aatggaacaa tacaaatcgt ttcttctcat      60

ataatccata atgtacactg tggattcatt ccatgtaata aaattaacgg agttatggac      120

gaagttgttt ataaaactgt tacagaaaat aacgaagctt gtaacaactg tggtgtttct      180

tatacttata aagtatatgg ggacatggaa ataatatgta aagctaaagc aaattag      237

<210>  14
<211>  255
<212>  DNA
<213>  microbiota

<400>  14
gccgaaccgg cggacaccgc gatttctgaa cggcgcgtgg aactttgcgg caactgcggc      60

ggacgaatgg taacttccac tacatgggga agctggtaca cagttgctca aatcaaatgc     120

acacaccata attatggtac cgatctccgt cagcagagag acggtacggc aacaacaaag     180

tgccagggct gcggacaggg ttacaccact tccaaatctc agacaagaat tgtttgccac     240

ggttacgatt cctaa                                                      255


<210>  15
<211>  435
<212>  DNA
<213>  microbiota

<400>  15
gcagcatttg ttttttccaa ctcgctcaaa cccgctaatg cctcaagcgc ggaaagcagc      60

cggctcctca tccatgtgaa ctcatttttt tcacagcttg ggcttaaacc gataagcgaa     120

aacctgcttc gcaaaacggc gcatttctgc gagttcggta tgctcggaat acttgcatcc     180

tccgcctgcg caatgttttc cggagcttat tctgccgcct ccctgccctc tctgcgccgc     240

cgtggctttt tcatctcctt tggcgtatct gtcgcgtgcg ccgtatgtga cgaaacgatc     300

cagtattttg ttcccggcag agcctgccgc gtgaccgata tgctcatcga ctccgccgga     360

gcactctgcg ggcttgctgc cgtacttgcg ttctgcgcgg caattcgcgt ccggcgccgg     420

cgccgccgga attga                                                      435


<210>  16
<211>  177
<212>  DNA
<213>  microbiota

<400>  16
ctggccgggc cgggttccgg ctgtcggttc acccccagct gttcgaccta cttcattcag      60

gccgtggaga ttcacggcgc gctcaagggc tcgctgatgg gcatctggcg cattctgcgc     120

tgcaatcctt ggggcggctg cggctatgat cccgtgccgc cgcgaaagcc ccgatga        177


<210>  17
<211>  135
<212>  DNA
<213>  microbiota

<400>  17
gcaaagctcg gcatggcggc gggcgcaatg ctggtgctcg ggctgctggc ggcgggtgcc      60

tccggcggca cgctcatcct tgcggcgctg gcactgtgtg ccgtgaccct gttgtgcggg     120

aggaaaaagc aatga                                                    135


<210>  18
<211>  228
<212>  DNA
<213>  microbiota

<400>  18
gtggaaaaga aaacagtgat tacgaaatgt gcaatcacag taaacgaata ccgcgaaaaa      60

gttgtcccgt caatgcgcaa aattcacgcc attgtcatat ttgtgagcta ctcaataaat     120

catttgtaca aaaattgtga gccagaacag ctattttcac cgggcagaaa aacaaaaaaa     180

ccgcccccgg cgacgtgccg aaagcggtta aatttacaat atttttaa                  228


<210>  19
<211>  291
<212>  DNA
<213>  microbiota

<400>  19
gaaataacac aaccatgtaa tcatgttaaa agtgattgga taatagataa agaagcaaca      60

tgtataggaa gttatgcctt ttacaattgt actagtttaa caagtataga aatttcaaca     120

agtgttacaa aaataaaata tcgtgcattt gctagttgtc gagctttaaa taatatatac     180

tacacaggaa cactaactca atggaatgaa ataagtaagg atactaattg gaattgggct     240

gcaccattaa attgtaaagt tatttgttta aatggtacat gttacttata a              291


<210>  20
<211>  297
<212>  DNA
<213>  microbiota

<400>  20
cttttggtct cggtatgcac cgccgccggg ctgcttgctg tggcgatgcg ccagatcgag      60

ccgctgctcg cttggctgcg cacactggag gtctactttc aggggcagag cccggcggtg     120

ctgctgcgcg ctctcggcat tgcgctggtc gcgcagtttg ccgccgacac ctgccgcgag     180

gccggtcttt gcgcggcctc caccgccatc gagctctgcg gccgtgtgct ggtgcttttg     240

caggctctgc cgctgctgcg gtcgctgctc ggctcctttg cggattattt gcagtaa        297

## Description

The present invention relates to microbiota-derived proteins, which are capable of inducing and/or enhancing secretion of IL-10 from human cells. The present invention also relates to nucleic acids encoding such proteins, cells expressing such proteins, respective pharmaceutical compositions and uses thereof.

The human intestinal microbiota (HIM) is a huge and complex community of 10¹⁴ bacteria which colonize the human gastrointestinal tract (GIT) and which is now considered as a hidden human organ. HIM has essential functions such as maintaining intestinal homeostasis, inhibiting the growth of pathogens, producing antimicrobial compounds and improving the intestinal barrier function. A number of evidences support the fact that several GI disorders including inflammatory bowel diseases (IBD), non-alcoholic steatohepatitis (NASH), alcoholic liver disease and even brain activity (gut brain axis) could be the result of an altered balance (dysbiosis) of the intestinal microbiota causing an abnormal production of particular metabolites and proteins. Over the last decade a huge amount of literature has described the identification of bacterial metabolites and their potential effects on human health (Levy M, Thaiss CA, Elinav E. Metabolites: messengers between the microbiota and the immune system. Genes Dev. 2016;30(14):1589-97; Guo CJ, Chang FY, Wyche TP, Backus KM, Acker TM, Funabashi M, Taketani M, Donia MS, Nayfach S, Pollard KS, Craik CS, Cravatt BF, Clardy J, Voigt CA, Fischbach MA. Discovery of Reactive Microbiota-Derived Metabolites that Inhibit Host Proteases. Cell. 2017;168(3):517-526.e18).

Currently, studies of interactions between bacterial proteins with human gut cells are emerging (Weigele BA, Orchard RC, Jimenez A, Cox GW, Alto NM. A systematic exploration of the interactions between bacterial effector proteins and host cell membranes. Nat Commun. 2017;8(1):532; Guven-Maiorov E, Tsai CJ, Nussinov R. Structural host-microbiota interaction networks. PLoS Comput Biol. 2017;13(10):e1005579). Microbial proteins may have the ability to interact with many human receptors, such as GPCRs, kinase receptors and transporters and may affect many different signalling pathways involved in immune surveillance, metabolism and cellular integrity. Molecular mimicry between human and microbial proteins have been proposed: *E. coli* protein ClpB, mimics the human alpha MSH, a neuropeptide playing a key role in signaling satiation (Breton J, Tennoune N, Lucas N, Francois M, Legrand R, Jacquemot J, Goichon A, Guerin C, Peltier J, Pestel-Caron M, Chan P, Vaudry D, do Rego JC, Liénard F, Pénicaud L, Fioramonti X, Ebenezer IS, Hökfelt T, Déchelotte P, Fetissov SO (2016) Gut Commensal E. coli Proteins Activate Host Satiety Pathways following Nutrient-Induced Bacterial Growth. Cell Metab. 2016 Feb 9;23(2):324-34); the *Helicobacter Pylori* CagA interacts with human tumor suppressor TP53BP2 >Buti L, Spooner E, Van der Veen AG, Rappuoli R, Covacci A, Ploegh HL. (2011) Helicobacter pylori cytotoxin-associated gene A (CagA) subverts the apoptosis-stimulating protein of p53 (ASPP2) tumor suppressor pathway of the host. Proc Natl Acad Sci USA. 2011 May 31;108(22):9238-43); SLPA from *Lactobacillus acidophilus,* is a DC-SIGN ligand functionally involved in the modulation of DCs and T cells functions (Konstantinov SR, Smidt H, de Vos WM, Bruijns SC, Singh SK, Valence F, Molle D, Lortal S, Altermann E, Klaenhammer TR, van Kooyk Y. (2008) S layer protein A of Lactobacillus acidophilus NCFM regulates immature dendritic cell and T cell functions. Proc Natl Acad Sci U S A. 2008 Dec 9;105(49):19474-9); FAp2 from *Fusobacterium nuc*/*eatum* has been shown to mediate Colorectal Adenocarcinoma enrichment by binding to tumor-expressed Gal-GalNAc, and to bind to the TIGIT receptor (Gur C, Ibrahim Y, Isaacson B, Yamin R, Abed J, Gamliel M, Enk J, Bar-On Y, Stanietsky-Kaynan N, Coppenhagen-Glazer S, Shussman N, Almogy G, Cuapio A5, Hofer E, Mevorach D, Tabib A, Ortenberg R, Markel G, Miklić K, Jonjic S, Brennan CA, Garrett WS, Bachrach G, Mandelboim O. (2015) Binding of the Fap2 protein of Fusobacterium nucleatum to human inhibitory receptor TIGIT protects tumors from immune cell attack. Immunity. 2015 Feb 17;42(2):344-355).

Thus, it is possible that proteins from commensal bacteria may have therapeutic applications by mimicking host proteins and interacting directly with the host cells and the immune system.

Interleukin-10 (IL-10) is a key immunoregulatory cytokine produced by various cell types, such as lymphocytes, monocytes, macrophages, mast cells and intestinal epithelial cells (Shouval DS, Biswas A, Goettel JA, McCann K, Conaway E, Redhu NS, Mascanfroni ID, Al Adham Z, Lavoie S, Ibourk M, Nguyen DD, Samsom JN, Escher JC, Somech R, Weiss B, Beier R, Conklin LS, Ebens CL, Santos FG, Ferreira AR, Sherlock M, Bhan AK, Müller W, Mora JR, Quintana FJ, Klein C, Muise AM, Horwitz BH, Snapper SB (2014) Interleukin-10 receptor signaling in innate immune cells regulates mucosal immune tolerance and anti-inflammatory macrophage function. Immunity. 2014 May 15;40(5):706-19) with the major functions of limiting mucosal immune responses and maintaining gut homeostasis.

IL-10 is often considered as the "prototype of anti-inflammatory cytokines" and it is known to downregulate the expression of Th1 cytokines, MHC class II antigens, and co-stimulatory molecules on macrophages. Its inhibitory action is exerted mainly against the most typical markers of inflammation such as IL-1, IL-6, TNF-α, GM-CSF and IFN-γ. The three major pro-inflammatory cytokines IL-1β, IL-6, and TNF-α are responsible for the onset and the maintenance of the inflammatory state in many diseases. While IL-10 is able to inhibit both the Th1-type and the Th2-type responses, the effect on Th1 subpopulation is predominant and IL-10 is considered as a promoter of the Th2 response (pleiotropic effect). In addition, IL-10 also enhances B cell survival, proliferation, antibody production as well as the production of anti-inflammatory factors including soluble TNF-α receptors and IL-1RA.

Polymorphisms in the IL10 locus confer the risk for ulcerative colitis and Crohn's disease (Franke A, Balschun T, Karlsen TH, Sventoraityte J, Nikolaus S, Mayr G, Domingues FS, Albrecht M, Nothnagel M, Ellinghaus D, Sina C, Onnie CM, Weersma RK, Stokkers PC, Wijmenga C, Gazouli M, Strachan D, McArdle WL, Vermeire S, Rutgeerts P, Rosenstiel P, Krawczak M, Vatn MH; IBSEN study group, Mathew CG, Schreiber S (2008) Sequence variants in IL10, ARPC2 and multiple other loci contribute to ulcerative colitis susceptibility. Nat Genet. 2008 Nov;40(11):1319-23; Franke A, McGovern DP, Barrett JC,... Parkes M. (2010) Genome-wide meta-analysis increases to 71 the number of confirmed Crohn's disease susceptibility loci. Nat Genet. 2010 Dec;42(12):1118-25), and mice and humans deficient in either IL-10 or IL-10 receptor (IL-10R) exhibit severe intestinal inflammation, a process dominated by a T helper type 1 and type 17 immune response and marked proinflammatory cytokines secretion (Begue B, Verdier J, Rieux-Laucat F, Goulet O, Morali A, Canioni D, Hugot JP, Daussy C, Verkarre V, Pigneur B, Fischer A, Klein C, Cerf-Bensussan N, Ruemmele FM. (2011) Defective IL10 signaling defining a subgroup of patients with inflammatory bowel disease. Am J Gastroenterol. Aug;106(8):1544-55; Moran CJ, Walters TD, Guo CH, Kugathasan S, Klein C, Turner D, Wolters VM, Bandsma RH, Mouzaki M, Zachos M, Langer JC, Cutz E, Benseler SM, Roifman CM, Silverberg MS, Griffiths AM, Snapper SB, Muise AM. (2013) IL-10R polymorphisms are associated with very-early-onset ulcerative colitis. Inflamm Bowel Dis. jan;19(1):115-23).

In view thereof, recombinant human IL-10 has emerged as candidate drug in acute and chronic inflammatory diseases, autoimmune disorders and allergic diseases, rejection of transplanted organs and graft-versus-host diseases after transplantation, and infectious diseases. However, studies with high-dose recombinant human IL-10 revealed several problems relating to the side effects of high-dose recombinant human IL-10 and the development of antibodies against recombinant human IL-10, restricting its usefulness in therapeutic applications (Fioranelli M. 2014, Twenty-five years of studies and trials for the therapeutic application of IL-10 immunomodulating properties. From high doses administration to low dose medicine new paradigm. J Integr Cardiol 1: DOI: 10.15761/JIC.1000102).

Accordingly, there is a need to provide increased IL-10 levels in therapeutic applications by means other than administration of high-dose recombinant human IL-10. In view thereof, it is the object of the present invention to provide compounds, which are capable of inducing secretion of (endogenous) IL-10.

This object is achieved by means of the subject-matter set out below, in particular in the items provided by the present invention and in the appended claims.

### ITEMS OF THE INVENTION

The present invention provides in particular the following items:
1. An isolated protein of non-pathogenic microbiota, or a fragment or sequence variant thereof, which is capable of inducing and/or enhancing IL-10 secretion from human cells.
2. The protein, or a fragment or sequence variant thereof, according to item 1, wherein the protein is a protein of the metasecretome of non-pathogenic microbiota.
3. The protein, or a fragment or sequence variant thereof, according to item 1 or 2, wherein the microbiota is selected from the group consisting of gastrointestinal tract microbiota, lung microbiota, saliva microbiota, seminal fluid microbiota, skin microbiota and vagina microbiota.
4. The protein, or a fragment or sequence variant thereof, according to any one of items 1 to 3, wherein the microbiota is gastrointestinal tract microbiota selected from gut microbiota and oral cavity microbiota.
5. The protein, or a fragment or sequence variant thereof, according to any one of items 1 to 4, wherein the protein is a bacterial protein, archaea protein, protist protein, fungi protein, virus protein and/or phage protein.
6. The protein, or a fragment or sequence variant thereof, according to any one of items 1 to 5, wherein the protein is a protein of human non-pathogenic microbiota.
7. The protein, or a fragment or sequence variant thereof, according to any one of items 1 to 6, wherein the protein is a bacterial protein, preferably of the human gastrointestinal tract microbiota metasecretome.
8. The protein, or a fragment or sequence variant thereof, according to any one of items 1 to 7, wherein the protein comprises at least two cysteine residues.
9. The protein, or a fragment or sequence variant thereof, according to any one of items 1 to 8, wherein the protein has a length of no more than 400 amino acids.
10. The protein, or a fragment or sequence variant thereof, according to any one of items 1 to 9, wherein the human cells are human immune cells, preferably PBMCs.
11. The protein, or a fragment or sequence variant thereof, according to any one of items 1 to 10, wherein the human cells are lymphocytes, preferably T lymphocytes.
12. The protein, or a fragment or sequence variant thereof, according to any one of items 1 to 11, wherein IL-10 secretion from human cells stimulated with said protein, or the fragment or sequence variant thereof, is higher than IL-10 secretion from human cells stimulated with an *E. coli* lysate.
13. The protein, or a fragment or sequence variant thereof, according to any one of items 1 to 12, wherein IL-10 secretion from human cells stimulated with said protein, or the fragment or sequence variant thereof, is higher than IL-10 secretion from human cells stimulated with PHA.
14. The protein, or a fragment or sequence variant thereof, according to any one of items 1 to 13, wherein IL-10 secretion from human cells stimulated with 0.1 µM of said protein, or the fragment or sequence variant thereof, is higher than IL-10 secretion from human cells stimulated with 10 µg/ml PHA.
15. The protein, or a fragment or sequence variant thereof, according to item 14, wherein IL-10 secretion from human cells stimulated with 0.025 µM of said protein, or the fragment or sequence variant thereof, is higher than IL-10 secretion from human cells stimulated with 10 µg/ml PHA.
16. The protein, or a fragment or sequence variant thereof, according to any one of items 1 to 15, wherein the protein, or the fragment or sequence variant thereof, comprises or consists of an amino acid sequence sharing at least 80% sequence identity with any one of SEQ ID NOs 1 to 10.
17. The protein, or a fragment or sequence variant thereof, according to any one of items 1 to 16, wherein the protein, or the fragment or sequence variant thereof, comprises or consists of an amino acid sequence sharing at least 80% sequence identity with SEQ ID NO: 1.
18. The protein according to any one of items 1 to 16, wherein the protein has an amino acid sequence according to SEQ ID NO: 1.
19. A nucleic acid comprising a polynucleotide encoding the protein, or the fragment or sequence variant thereof, according to any one of items 1 to 18.
20. The nucleic acid according to item 19, wherein the nucleic acid is a DNA molecule or an RNA molecule; preferably selected from genomic DNA; cDNA; siRNA; rRNA; mRNA; antisense DNA; antisense RNA; ribozyme; complementary RNA and/or DNA sequences; RNA and/or DNA sequences with or without expression elements, regulatory elements, and/or promoters; a vector; and combinations thereof.
21. The nucleic acid according to item 19 or 20, wherein the nucleic acid sequence of the polynucleotide encoding the microbiota protein, or the fragment or sequence variant thereof, shares at least 80% sequence identity with any one of SEQ ID NOs 10 to 20.
22. The nucleic acid according to any one of items 19 to 21, wherein the polynucleotide encoding the protein, or the fragment or sequence variant thereof, according to any one of items 1 to 18, is codon-optimized for expression by prokaryotic cells, preferably bacteria.
23. An expression cassette comprising a polynucleotide encoding the protein, or the fragment or sequence variant thereof, according to any one of items 1 to 18 and, operably linked thereto, a regulatory element, preferably for expression in a prokaryotic cell, such as a bacterium.
24. A vector comprising the nucleic acid according to any one of items 19 to 22 or the expression cassette according to item 19.
25. A (host) cell expressing the microbiota protein, or the fragment or sequence variant thereof, according to any one of items 1 to 18; or comprising the nucleic acid according to any one of items 19 to 22, the expression cassette according to item 23, or the vector according to item 24.
26. The (host) cell according to item 25, wherein the (host) cell is a bacterium, preferably a genetically engineered bacterium.
27. A genetically engineered bacterium capable of inducing and/or enhancing IL-10 secretion from a human cell, the bacterium comprising the nucleic acid according to any one of items 19 to 22, the expression cassette according to item 23, or the vector according to item 24.
28. A genetically engineered bacterium (over)expressing the protein, or the fragment or sequence variant thereof, according to any one of items 1 to 18.
29. A culture medium comprising the protein, or the fragment or sequence variant thereof, according to any one of items 1 to 18, the (host) cell according to item 25 or 26, or the bacterium according to item 27 or 28.
30. The culture medium according to item 29 further comprising a (self) antigen.
31. An isolated human cell cultured with the culture medium according to item 29 or 30.
32. The cell according to item 31, wherein the cell is a human immune cell, preferably a PBMC.
33. The cell according to item 31 or 32, wherein the cell is a monocyte, a macrophage, a dendritic cell or a lymphocyte, preferably a T lymphocyte.
34. A pharmaceutical composition comprising the protein, or the fragment or sequence variant thereof, according to any one of items 1 to 18, the nucleic acid according to any one of items 19 to 22, the vector according to item 24, the cell according to item 25 or 26, the bacterium according to item 27 or 28, or the human cell according to any one of items 31 to 33 and, optionally, a pharmaceutically acceptable excipient or carrier.
35. The protein, or the fragment or sequence variant thereof, according to any one of items 1 to 18, the nucleic acid according to any one of items 19 to 22, the vector according to item 24, the cell according to item 25 or 26, the bacterium according to item 27 or 28, the human cell according to any one of items 31 to 33, or the pharmaceutical composition according to item 34 for use in medicine.
36. The protein, or the fragment or sequence variant thereof, the nucleic acid, the vector, the cell, the bacterium, the human cell or the pharmaceutical composition for use according to item 35 in treating an inflammatory disease or an autoimmune disorder.
37. A method for reducing, treating, alleviating symptoms of or ameliorating an inflammatory disease or an autoimmune disorder in a subject, comprising the step of administering to said subject the protein, or the fragment or sequence variant thereof, according to any one of items 1 to 18, the nucleic acid according to any one of items 19 to 22, the vector according to item 24, the cell according to item 25 or 26, the bacterium according to item 27 or 28, the human cell according to any one of items 31 to 33, or the pharmaceutical composition according to item 34.
38. A method for inducing and/or enhancing IL-10 secretion in a subject, comprising the step of administering to said subject the protein, or the fragment or sequence variant thereof, according to any one of items 1 to 18, the nucleic acid according to any one of items 19 to 22, the vector according to item 24, the cell according to item 25 or 26, the bacterium according to item 27 or 28, or the pharmaceutical composition according to item 34.
39. A method for inducing tolerance in a subject, comprising the step of administering to said subject the protein, or the fragment or sequence variant thereof, according to any one of items 1 to 18, the nucleic acid according to any one of items 19 to 22, the vector according to item 24, the cell according to item 25 or 26, the bacterium according to item 27 or 28, the human cell according to any one of items 31 to 33, or the pharmaceutical composition according to item 34.

The invention, and in particular the items outlined above, are described in more detail below.

### DEFINITIONS

Unless otherwise defined herein, scientific and technical terms used in the present application shall have the meanings that are commonly understood by those of ordinary skill in the art. Further, unless otherwise required by context, nomenclatures used herein, and techniques of cell and tissue culture are those well-known and commonly used in the art.

Such techniques are fully explained in the literature, such as Owen et al. (Kuby Immunology, 7th, edition, 2013 - W. H. Freeman) and Sambrook et al. (Molecular cloning: A laboratory manual 4th edition, Cold Spring Harbor Laboratory Press - Cold Spring Harbor, NY, USA, 2012).

Nevertheless, with respect to the use of different terms throughout the current specification, the following definitions more particularly apply.

The term "(poly)peptide" as used herein refers to a peptide and/or to a polypeptide. The terms "peptide", "polypeptide", "protein" and variations of these terms refer to peptides, oligopeptides, polypeptides, or proteins comprising at least two amino acids joined to each other preferably by a normal peptide bond, or, alternatively, by a modified peptide bond, such as for example in the cases of isosteric peptides. In particular, the terms "peptide", "polypeptide" and "protein" refer to a sequential chain of amino acids of any length linked together via peptide bonds (-NHCO-). Peptides, polypeptides and proteins can play a structural and/or functional role in a cell *in vitro* and/or *in vivo.* The terms "peptide", "polypeptide", "protein" preferably encompass amino acids chains in size ranging from 2 to at least about 1000 amino acid residues. The term "peptide" preferably encompasses herein amino acid chains in size of less than about 30 amino acids, while the terms "polypeptide" and "protein" preferably encompass amino acid chains in size of at least 30 amino acids. The terms "polypeptide" and "protein" are used herein interchangeably. In some embodiments, the terms "peptide", "polypeptide", "protein" also include "peptidomimetics" which are defined as peptide analogs containing non-peptidic structural elements, which peptides are capable of mimicking or antagonizing the biological action(s) of a natural parent peptide. A peptidomimetic lacks classical peptide characteristics such as enzymatically scissile peptide bonds. In particular, a peptide, polypeptide or protein can comprise amino acids other than the 20 amino acids defined by the genetic code in addition to these amino acids, or it can be composed of amino acids other than the 20 amino acids defined by the genetic code. In particular, a peptide, polypeptide or protein in the context of the present invention can equally be composed of amino acids modified by natural processes, such as post-translational maturation processes or by chemical processes, which are well known to a person skilled in the art. Such modifications are fully detailed in the literature. These modifications can appear anywhere in the polypeptide: in the peptide skeleton, in the amino acid chain or even at the carboxy- or amino-terminal ends. In particular, a peptide or polypeptide can be branched following an ubiquitination or be cyclic with or without branching. This type of modification can be the result of natural or synthetic post-translational processes that are well known to a person skilled in the art. The terms "peptide", "polypeptide", "protein" in the context of the present invention in particular also include modified peptides, polypeptides and proteins. For example, peptide, polypeptide or protein modifications can include acetylation, acylation, ADP-ribosylation, amidation, covalent fixation of a nucleotide or of a nucleotide derivative, covalent fixation of a lipid or of a lipidic derivative, the covalent fixation of a phosphatidylinositol, covalent or non-covalent cross-linking, cyclization, disulfide bond formation, demethylation, glycosylation including pegylation, hydroxylation, iodization, methylation, myristoylation, oxidation, proteolytic processes, phosphorylation, prenylation, racemization, seneloylation, sulfatation, amino acid addition such as arginylation or ubiquitination. Such modifications are fully detailed in the literature (Proteins Structure and Molecular Properties (1993) 2nd Ed., T. E. Creighton, New York ; Post-translational Covalent Modifications of Proteins (1983) B. C. Johnson, Ed., Academic Press, New York ; Seifter et al. (1990) Analysis for protein modifications and nonprotein cofactors, Meth. Enzymol. 182: 626-646 and Rattan et al., (1992) Protein Synthesis: Post-translational Modifications and Aging, Ann NY Acad Sci, 663: 48-62). Accordingly, the terms "peptide", "polypeptide", "protein" preferably include for example lipopeptides, lipoproteins, glycopeptides, glycoproteins and the like.

Preferably, a (poly)peptide or protein is a "classical" (poly)peptide or protein, whereby a "classical" (poly)peptide or protein is typically composed of amino acids selected from the 20 amino acids defined by the genetic code, linked to each other by a normal peptide bond.

In general, a protein or (poly)peptide may be of any length. Preferably, the length of the proteins of the inventions, or the sequence variant thereof, does not exceed 500 amino acids. For example, the maximum length of the protein according to the present invention (or the fragment or sequence variant thereof) may be 400 or 350 amino acids. More preferably, the maximum length of the microbiota protein according to the present invention (or the fragment or sequence variant thereof) does not exceed 250 amino acids, e.g., not more than 240, 230, 220, 210, 200, 190, 180, 170, 160, 150, 140, 130, 120, 110, 100, 95, 90, 85, 80, 75, 70, 65, 60, 55, 50, 45, 40, 35, 30, 29, 28, 27, 26, 25, 24, 23, 22, 21, or 20 amino acids. In particular, the length of the protein is assessed in the "mature" protein (in its functional state), i.e. "additional" pre-protein sequences, like signal peptides, which are removed in the mature/functional protein, are usually not considered for the length of the protein.

As well-known in the art, peptides, polypeptides and proteins can be encoded by nucleic acids. The terms "nucleic acid", "nucleic acid molecule", "nucleic acid sequence", "polynucleotide", "nucleotide sequence" are used herein interchangeable and refer to a precise succession of natural nucleotides (e.g., A, T, G, C and U), or synthetic nucleotides, i.e. to a chain of at least two nucleotides. In particular, the terms "nucleic acid", "nucleic acid molecule", "nucleic acid sequence", "polynucleotide", "nucleotide sequence" refer to DNA or RNA. Nucleic acids preferably comprise single stranded, double stranded or partially double stranded DNA or RNA, preferably selected from genomic DNA, cDNA, ribosomal DNA, and the transcription product of said DNA, such as RNA. Preferred examples of nucleic acids include rRNA, mRNA; antisense DNA, antisense RNA; complimentary RNA and/or DNA sequences, ribozyme, (complementary) RNA/DNA sequences with or without expression elements, a vector; a mini-gene, gene fragments, regulatory elements, promoters, and combinations thereof. Further preferred examples of nucleic acid (molecules) and/or polynucleotides include, e.g., a recombinant polynucleotide, a vector, an oligonucleotide, an RNA molecule such as an rRNA, an mRNA, or a tRNA, or a DNA molecule as described above. It is thus preferred that the nucleic acid (molecule) is a DNA molecule or an RNA molecule; preferably selected from genomic DNA; cDNA; rRNA; mRNA; antisense DNA; antisense RNA; complementary RNA and/or DNA sequences; RNA and/or DNA sequences with or without expression elements, regulatory elements, and/or promoters; a vector; and combinations thereof. It is within the skill of the person in the art to determine nucleotide sequences which can encode a specific amino acid sequence.

The proteins and/or nucleic acids according to the invention may be prepared by any known method in the art including, but not limited to, any synthetic method, any recombinant method, any *ex vivo* generation method and the like, and any combination thereof. Such techniques are well-known in the art.

In general, the term "sequence variant", as used herein, i.e. throughout the present application, refers to a sequence which is similar (meaning in particular at least 50% sequence identity, see below), but not (100%) identical, to a reference sequence (such as the microbiota proteins or fragment thereof). Accordingly, a sequence variant contains at least one alteration in comparison to a reference sequence, e.g. the microbiota protein or a fragment thereof. For example, in a sequence variant one or more of the amino acids or nucleotides of the reference sequence is deleted or substituted, or one or more amino acids or nucleotides are inserted into or added to the sequence of the reference sequence. Therefore, the "sequence variant" is similar, but contains at least one alteration, in comparison to its reference sequence, such as a microbiota protein sequence. Preferably, a sequence variant shares, in particular over the whole length of the sequence, at least 70%, preferably at least 75%, more preferably at least 80%, even more preferably at least 85%, still more preferably at least 90%, particularly preferably at least 95% and most preferably at least 98% or 99% sequence identity with a reference sequence (such as the microbiota protein of the invention). A sequence variant may preserve the specific function of the reference sequence. In the context of the present invention, this function may be the functionality of inducing and/or enhancing IL-10 secretion (from human cells).

The term "sequence variant" includes nucleotide sequence variants and amino acid sequence variants. For example, an amino acid sequence variant has an altered sequence in which one or more of the amino acids is deleted or substituted in comparison to the reference sequence, or one or more amino acids are inserted or added in comparison to the reference amino acid sequence. As a result of the alterations, the amino acid sequence variant has an amino acid sequence which is at least 50%, e.g. at least 70%, preferably at least 75%, more preferably at least 80%, even more preferably at least 85%, still more preferably at least 90%, particularly preferably at least 95% and most preferably at least 98% or 99% identical to the reference sequence. For example, variant sequences which are at least 90% identical have no more than 10 alterations (i.e. any combination of deletions, insertions or substitutions) per 100 amino acids of the reference sequence.

In the context of the present invention, an amino acid sequence "sharing a sequence identity" of at least, for example, 70% to a query (reference) amino acid sequence of the present invention, is intended to mean that the sequence of the subject amino acid sequence is identical to the query sequence except that the subject amino acid sequence may include up to three amino acid alterations per each 10 amino acids of the query amino acid sequence. In other words, to obtain an amino acid sequence having a sequence of at least 70% identity to a query amino acid sequence, up to 30% (3 of 10) of the amino acid residues in the subject sequence may be inserted or substituted with another amino acid or deleted, preferably within the above definitions of variants or fragments. The same, of course, also applies similarly to nucleic acid sequences.

Amino acid substitutions are preferably conservative amino acid substitutions. Examples of conservative substitutions include substitution of one aliphatic residue for another, such as Ile, Val, Leu, or Ala for one another; or substitutions of one polar residue for another, such as between Lys and Arg; Glu and Asp; or Gln and Asn. Other such conservative substitutions, for example, substitutions of entire regions having similar hydrophobicity properties, are well known (Kyte and Doolittle, 1982, J. Mol. Biol. 157(1):105- 132). Examples of conservative amino acid substitutions are presented in Table 1 below:

**(Table 1)**

| **Original residues** | **Examples of substitutions** |
|---|---|
| Ala (A) | Val, Leu, Ile, Gly |
| Arg (R) | His, Lys |
| Asn (N) | Gln |
| Asp (D) | Glu |
| Cys (C) | Ser |
| Gln (Q) | Asn |
| Glu (E) | Asp |
| Gly (G) | Pro, Ala |
| His (H) | Lys, Arg |
| Ile (I) | Leu, Val, Met, Ala, Phe |
| Leu (L) | Ile, Val, Met, Ala, Phe |
| Lys (K) | Arg, His |
| Met (M) | Leu, Ile, Phe |
| Phe (F) | Leu, Val, Ile, Tyr, Trp, Met |
| Pro (P) | Ala, Gly |
| Ser (S) | Thr |
| Thr (T) | Ser |
| Trp (W) | Tyr, Phe |
| Tyr (Y) | Trp, Phe |
| Original residues | Examples of substitutions |
| Val (V) | Ile, Met, Leu, Phe, Ala |

For (amino acid or nucleic acid) sequences without exact correspondence, a "% identity" of a first sequence (e.g., the sequence variant) may be determined with respect to a second sequence (e.g., the reference sequence). In general, the two sequences to be compared may be aligned to give a maximum correlation between the sequences. This may include inserting "gaps" in either one or both sequences, to enhance the degree of alignment. A % identity may then be determined over the whole length of each of the sequences being compared (so-called "global alignment"), that is particularly suitable for sequences of the same or similar length, or over shorter, defined lengths (so-called "local alignment"), that is more suitable for sequences of unequal length.

Methods for comparing the identity (sometimes also referred to as "similarity" or "homology") of two or more sequences are well known in the art. The percentage to which two (or more) sequences are identical can e.g. be determined using a mathematical algorithm. A preferred, but not limiting, example of a mathematical algorithm which can be used is the algorithm of Karlin et al. (1993), PNAS USA, 90:5873-5877. Such an algorithm is integrated in the BLAST family of programs, e.g. BLAST or NBLAST program (see also Altschul et al., 1990, J. Mol. Biol. 215, 403-410 or Altschul et a/. (1997), Nucleic Acids Res, 25:3389-3402), accessible through the home page of the NCBI at world wide web site ncbi.nlm.nih.gov) and FASTA (Pearson (1990), Methods Enzymol. 183, 63-98; Pearson and Lipman (1988), Proc. Natl. Acad. Sci. U. S. A 85, 2444-2448.). Sequences which are identical to other sequences to a certain extent can be identified by these programmes. Furthermore, programs available in the Wisconsin Sequence Analysis Package, version 9.1 (Devereux et al., 1984, Nucleic Acids Res., 387-395), for example the programs BESTFIT and GAP, may be used to determine the % identity between two polynucleotides and the % identity and the % homology or identity between two polypeptide sequences. BESTFIT uses the "local homology" algorithm of (Smith and Waterman (1981), J. Mol. Biol. 147, 195-197.) and finds the best single region of similarity between two sequences.

By "pharmaceutically acceptable excipient", it is meant herein a compound of pharmaceutical grade which improves the delivery, stability or bioavailability of an active agent, and can be metabolized by, and is non-toxic to, a subject to whom it is administered. Preferred excipients according to the invention include any of the excipients commonly used in pharmaceutical products, such as, for example, water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition. Pharmaceutically acceptable excipients may further comprise minor amounts of auxiliary substances such as wetting or emulsifying agents, or preservatives.

According to the different aspects and embodiments of the invention described herein, a "subject" or "host" preferably refers to a mammal, and most preferably to a human being.

As used herein, the expression "treatment of a disease" generally refers to ameliorating, reducing, preventing and/or treating a disease or condition or to reducing or preventing the occurrence or the risk of developing a disease or condition. Accordingly, the expression "treatment of a disease" refers to prophylactic as well as therapeutic settings. Prophylactic settings generally mean to avoid or minimize the onset, development or recurrence of a disease or condition before its onset or after it was "healed", while therapeutic settings usually encompass reducing, ameliorating or curing a disease or condition (or symptoms of a disease or condition) after its onset. In particular, the term "preventing" encompasses "reducing the likelihood of occurrence of" or "reducing the likelihood of reoccurrence".

An "effective amount" or "effective dose" as used herein is an amount which provides the desired effect. For therapeutic purposes, an effective amount is an amount sufficient to provide a beneficial or desired clinical result. The preferred effective amount for a given application can be easily determined by the skilled person taking into consideration, for example, the size, age, weight of the subject, the type of disease/disorder to be prevented or treated, and the amount of time since the disease/disorder began. In the context of the present invention, in terms of treatment, an effective amount of the composition is usually an amount that is sufficient to enhance and/or induce IL-10 secretion from human cells.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of". The term "comprising" thus encompasses "including" as well as "consisting" *e.g*., a composition "comprising" X may consist exclusively of X or may include something additional *e.g.*, X + Y.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

The word "substantially" does not exclude "completely" *e.g*., a composition which is "substantially free" from Y may be completely free from Y. Where necessary, the word "substantially" may be omitted from the definition of the invention.

The term "about" in relation to a numerical value x means x ± 10%.

Additional definitions are provided throughout the specification.

The present invention may be understood more readily by reference to the following detailed description, including preferred embodiments of the invention, and examples included herein.

### DETAILED DESCRIPTION

Although the present invention is described in detail below, it is to be understood that this invention is not limited to the particular methodologies, protocols and reagents described herein as these may vary. It is also to be understood that the terminology used herein is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

### Microbiota-derived protein inducing IL - 10 secretion from human cells

In a first aspect, the present invention provides an isolated protein of non-pathogenic microbiota, or a fragment or sequence variant thereof, which is capable of inducing and/or enhancing IL-10 secretion from human cells.

A protein of non-pathogenic microbiota (also referred to herein as "microbiota protein") is a protein of non-pathogenic microbiota origin. While a protein of non-pathogenic microbiota is originally expressed by non-pathogenic microbiota, it is understood that the present invention also encompasses non-pathogenic microbiota proteins produced by other means, e.g. recombinantly. As used herein, the term "recombinant" is intended to refer to such compounds, e.g. proteins, that are prepared, expressed, created or isolated by recombinant means, such as proteins isolated from a heterologous host cell (which does not express said protein in nature) or proteins expressed using a recombinant expression vector transfected into a host cell. Thus, recombinant proteins are proteins that are prepared, expressed, created or isolated by recombinant means, such as proteins isolated from a host cell transfected to express the protein, proteins isolated from a recombinant protein library, and proteins prepared, expressed, created or isolated by any other means that involve splicing of the respective gene sequence encoding the protein into other DNA sequences (distinct from the DNA sequences flanking said gene in nature). Recombinant proteins may have glycosylation patterns, which do not occur in nature. In some embodiments, the microbiota protein is a recombinant microbiota protein.

The term "microbiota", as used herein, refers to commensal, symbiotic and pathogenic microorganisms found in and on all multicellular organisms studied to date from plants to animals. In particular, microbiota have been found to be crucial for immunologic, hormonal and metabolic homeostasis of their host. In particular, the microbiota is non-pathogenic. In other words, the microbiota are usually not (capable of) causing a disease in the host and/or they are preferably not harmful to the host. Preferably, the microbiota are commensal or symbiotic microbiota (i.e., the interaction between microbiota and their host is commensal or symbiotic). Microbiota include bacteria, archaea, protists, fungi, viruses and phages. Accordingly, the microbiota protein may be a bacterial protein, an archaea protein, a protist protein, a fungi protein, a virus protein and/or a phage protein. Preferably, the human microbiota protein is a bacterial protein or an archaea protein. More preferably, the microbiota protein is a bacterial protein.

Anatomically, microbiota reside on or within any of a number of tissues and biofluids, including the gastrointestinal tract, in particular the gut (and the oral cavity, in particular the oral mucosa), skin, conjunctiva, mammary glands, vagina, placenta, seminal fluid, uterus, ovarian follicles, lung and saliva. Accordingly, the microbiota may be selected from the group consisting of gastrointestinal tract microbiota, lung microbiota, saliva microbiota, seminal fluid microbiota, skin microbiota and vagina microbiota. In some embodiments, the microbiota are gastrointestinal tract microbiota selected from gut microbiota and oral cavity microbiota. Accordingly, the protein of the human microbiota metasecretome may be a microbial protein of microbiota residing in the human gastrointestinal tract, i.e. by "human gastrointestinal tract microbiota". This includes, for example, proteins expressed by microbiota residing in the gut and/or in the oral cavity of a human. In other embodiments, the protein of the human microbiota protein is a microbial protein, which is expressed by microbiota residing in tissues and body fluids other than (outside the) gastrointestinal tract, e.g. in the skin or the genital system.

While microbiota can be found in and on many multicellular organisms (all multicellular organisms studied to date from plants to animals), microbiota found in and on mammals are preferred. Mammals contemplated by the present invention include for example human, primates, domesticated animals such as cattle, sheep, pigs, horses, laboratory rodents and the like. Microbiota found in and on humans are more preferred. Such microbiota are referred to herein as "mammalian microbiota" or "human microbiota" (wherein the term mammalian/human refers specifically to the localization/residence of the microbiota). Accordingly, the protein may be a human microbiota protein.

Preferably, the microbiota protein is a protein of the metasecretome of microbiota, more preferably of the human gastrointestinal tract microbiota metasecretome, such as the human gut microbiota metasecretome, i.e. a microbial protein of microbiota residing in the human gastrointestinal tract, in particular in the human gut. Even more preferably, the protein is a protein of the human gut bacteria metasecretome, i.e. a bacterial protein (originally) expressed by bacteria residing in the human gut.

As used herein, the term "metasecretome" refers to a collection of secreted proteins, outer surface proteins and transmembrane proteins from environmental (microbial) communities (microbiome), such as the microbial communities residing on or within human tissues and/or biofluids as described above, for example in the human gastrointestinal tract. Accordingly, the expression "human microbiota metasecretome" refers to a collection of secreted proteins, outer surface proteins and transmembrane proteins from microbiota residing in (or on) human hosts. Without being bound to any theory it is assumed that the metasecretome open reading frames (ORFs) comprises only 10% to 30% of total metagenome. The term "secretome" refers to a collection of proteins consisting of transmembrane proteins (TM), outer surface proteins and proteins secreted by cells into the extracellular milieu/space. Preferably, the metasecretome protein is a secreted protein. In other words, in some embodiments, the protein is released from the microbiota (expressing the protein), in particular into the extracellular milieu/space, and not attached to said microbiota (or its outer surface).

The present invention also encompasses fragments of microbiota proteins. Such a fragment typically comprises or consists of a protein domain responsible for inducing and/or enhancing IL-10 secretion from human cells. However, usually a protein comprises distinct domains mediating distinct functions. Functionalities other than inducing and/or enhancing IL-10 secretion from human cells may be not required in a protein of the invention and may, thus, be omitted. Accordingly, similarly as the microbiota protein of the invention, also the fragment thereof is capable of inducing and/or enhancing IL-10 secretion from human cells.

As used herein, a "fragment" of the microbiota protein comprises at least 10 consecutive amino acids of the microbiota protein, preferably at least 15 consecutive amino acids of the microbiota protein, more preferably at least 20 consecutive amino acids of the microbiota protein, even more preferably at least 25 consecutive amino acids of the microbiota protein, still more preferably at least 30 consecutive amino acids of the microbiota protein and particularly preferably at least 35 consecutive amino acids of the microbiota protein. Accordingly, the fragment of the microbiota protein has a length of at least 10 amino acids, preferably at least 15 amino acids, more preferably at least 20 amino acids, even more preferably at least 25 amino acids, still more preferably at least 30 amino acids and most preferably at least 35 amino acids. For example, the fragment of the microbiota protein may comprise 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300 or more consecutive amino acids of the microbiota protein. It is understood that the fragment of the microbiota protein is in any case shorter than the (full-length) microbiota protein (and has a minimum length of 10 amino acids).

For example, a fragment of a microbiota protein may be at least 1%, 2%, 3%, 4%, 5%, 6%, 7%, 8%, 9%, 10%, 11%, 12%, 13%, 14%, 15%, 16%, 17%, 18%, 19%, 20% or more, e.g. 25%, 30%, 35%, 40%, 45%, 50%, 55%, 60%, 65%, 70%, 75%, 80%, 85%, 90%, 95% or more, shorter than the full-length microbiota protein (representing the "reference" with 100%).

The present invention also encompasses sequence variants of microbiota proteins and fragments thereof. A "sequence variant" is defined as outlined above. Preferably, a sequence variant shares, in particular over the whole length of the sequence, at least 70%, preferably at least 75%, more preferably at least 80%, even more preferably at least 85%, still more preferably at least 90%, particularly preferably at least 95% and most preferably at least 98% or 99% sequence identity with the (full-length) microbiota protein or a fragment thereof as described above.

A sequence variant may preserve the specific function of the reference sequence. In the context of the present invention, the sequence variant maintains the functionality of inducing and/or enhancing IL-10 secretion (from human cells).

In view of their functionality to induce and/or enhance IL-10 secretion from human cells, the microbiota protein, or the fragment or sequence variant thereof, of the present invention is also referred to as "secretagogue". In general, the term "secretagogue" refers to substances causing other substances to be secreted.

Various human cells were described to secrete IL-10, including immune cells and non-immune cells (e.g., epithelial and neuronal cells). Preferably, the microbiota protein, or the fragment or sequence variant thereof, induces and/or enhances IL-10 secretion from human immune cells, e.g. peripheral blood mononuclear cells (PBMCs). For example, various blood cell types, including lymphocytes, monocytes, dendritic cells and granulocytes were reported to produce IL-10. In some embodiments, the microbiota protein, or the fragment or sequence variant thereof, induces and/or enhances IL-10 secretion from lymphocytes, e.g. T lymphocytes.

The skilled person is aware of various methods to identify whether a microbiota protein, or a fragment or sequence variant thereof, is capable of inducing and/or enhancing IL-10 secretion from human cells. For example, human cells capable of IL-10 secretion (as outlined above, e.g. PBMCs, lymphocytes, monocytes, dendritic cells or granulocytes) may be cultured in presence and absence of the microbiota protein, or the fragment or sequence variant thereof, and the IL-10 secretion in both cases (presence/absence) may be compared. It is understood that for such a comparative purpose, the human cells used as well as the other experimental conditions (except for the presence/absence of the microbiota protein, or the fragment or sequence variant thereof) are usually identical. In particular for assessing induction of IL-10 secretion, the culture medium may be devoid of IL-10 inducing compounds (other than the microbiota protein, or the fragment or sequence variant thereof, for the respective experimental group). In the cells cultured in absence of the microbiota protein, or the fragment or sequence variant thereof, of the invention, a control compound may be used. For example, such cells may be cultured in the presence of a negative control (not IL-10 inducing) or of positive control (known to be IL-10 inducing). Kits for determining IL-10 secretion are commercially available, e.g. "IL-10 Human ELISA Kit" (Invitrogen); "Human IL-10 Quantikine ELISA Kit D1000B" (R&D Systems); "Simoa® IL-10 Advantage Kit" (Quanterix); "IL-10 Secretion Assay" (Miltenyi Biotec); "Human IL10 kit" (Cisbio); "Human IL-10 ELISA Kit" (Abcam) etc. A specific example for determining whether a microbiota protein, or a fragment or sequence variant thereof, is capable of inducing and/or enhancing IL-10 secretion from human cells, is provided in the example section of the present specification.

In general, a microbiota protein, or a fragment or sequence variant thereof, is considered to be capable of inducing and/or enhancing IL-10 secretion from human cells, if human cells increase more IL-10, when cultured in the presence of said microbiota protein, or said fragment or sequence variant thereof, as compared to the same human cell type cultured under the same conditions, but in the absence of said microbiota protein, or said fragment or sequence variant thereof (wherein in the latter case a negative control may be included).

In some embodiments, secretion of IL-10 from human cells cultured in the presence of the microbiota protein, or the fragment or sequence variant thereof, of the invention is at least 1.5-fold, 2-fold, 2.5-fold, 3-fold, 4-fold, 5-fold, 6-fold, 7-fold, 8-fold, 9-fold or 10-fold higher than the IL-10 level produced by the same type of human cells cultured in the absence of the microbiota protein, or the fragment or sequence variant thereof, of the invention and/or cultured with a control compound.

In some embodiments, IL-10 secretion from human cells stimulated with the microbiota protein, or the fragment or sequence variant thereof, of the present invention is higher than IL-10 secretion from the same type of human cells stimulated with an *E. coli* lysate. The *E. coli* lysate may be prepared according to the method described in Zubay G. In vitro synthesis of protein in microbial systems. Annu Rev Genet. 1973;7:267-87. An *E. coli* strain with low exonuclease activity may be used and growth conditions may be optimized to allow optimum protein expression from linear and plasmid templates.

In some embodiments, IL-10 secretion from human cells stimulated with the microbiota protein, or the fragment or sequence variant thereof, of the present invention is higher than IL-10 secretion from the same type of human cells stimulated with phytohemagglutinin (PHA). Phytohemagglutinin (PHA) is an extract from the red kidney bean (Phaseolus vulgaris) that contains potent cell-agglutinating and mitogenic properties. PHA is known to induce IL-10 secretion from human cells and, thus, it is often used as positive control. In certain embodiments, IL-10 secretion from human cells stimulated with 0.1 µM of the microbiota protein, or the fragment or sequence variant thereof, of the present invention is higher than IL-10 secretion from the same type of human cells stimulated with 10 µg/ml PHA. In certain instances, IL-10 secretion from human cells stimulated with concentrations as low as 0.025 µM of the microbiota protein, or the fragment or sequence variant thereof, of the present invention is still higher than IL-10 secretion from the same type of human cells stimulated with 10 µg/ml PHA.

In some embodiments, the microbiota protein (or the fragment or sequence variant thereof) has a length of 20 - 350 amino acids, preferably a length of 20 - 300 amino acids, more preferably a length of 20 - 250 amino acids, even more preferably a length of 20 - 200 amino acids and still more preferably 20 - 150 amino acids, for example a length of 20 to 40, 50, 60, 70, 80, 90, 100, 110, 120, 130 or 140 amino acids. In particular, the length of the protein is assessed in the "mature" protein (in its functional state), i.e. "additional" pre-protein sequences, like signal peptides, which are removed in the mature/functional protein, are usually not considered for the length of the protein.

The microbiota protein (or the fragment or sequence variant thereof) may comprise at least two cysteine residues (which may form a cysteine pair). Cysteine residues stabilize the protein in the correct conformation. In some embodiments, the protein comprises 3, 4, 5, 6, 7, 8, 9, 10 or more cysteine residues. For example, the protein (or the fragment or sequence variant thereof) may comprise two or three cysteine residues (e.g., forming one cysteine pair), more preferably four or five cysteine residues (e.g., forming two cysteine pairs), even more preferably six or seven cysteine residues (e.g., forming three cysteine pairs), and still more preferably eight or nine cysteine residues (e.g., forming four cysteine pairs). Similarly as the length, the cysteine content is usually assessed in the "mature" protein (in its functional state), i.e. "additional" pre-protein sequences, like signal peptides, which are removed in the mature/functional protein, are usually not considered for the cysteine content of the protein.

The cysteine content of the protein (or the fragment or sequence variant thereof) may also be calculated in view of the length of the protein. For example, the protein (or the fragment or sequence variant thereof) has a cysteine content of at least 3%. More preferably, the protein (or the fragment or sequence variant thereof) has a cysteine content of at least 4%. Even more preferably, the protein (or the fragment or sequence variant thereof) has a cysteine content of at least 5%. Still more preferably, the protein (or the fragment or sequence variant thereof) has a cysteine content of at least 5%. Most preferably, the protein (or the fragment or sequence variant thereof) may have a cysteine content of about 6 - 8%, e.g. about 6.5 - 7%.

Preferably, the human microbiota protein (or the fragment or sequence variant thereof) has a length of 20 - 200 amino acids and comprises at least two cysteine residues (e.g., forming one cysteine pair). For example, the microbiota protein (or the fragment or sequence variant thereof) may have a length of 50 - 150 amino acids and may comprise at least four cysteine residues (e.g., forming two cysteine pairs); or the microbiota protein (or the fragment or sequence variant thereof) may have a length of 75 - 150 amino acids and may comprise at least six cysteine residues (e.g., forming three cysteine pairs). More preferably, the microbiota protein (or the fragment or sequence variant thereof) has (i) a length of 20 - 100 amino acids and comprises at least four cysteine residues (e.g., forming two cysteine pairs); (ii) a length of 50 - 150 amino acids and comprises at least six cysteine residues (e.g., forming three cysteine pairs); or (iii) a length of 75 - 200 amino acids and comprises at least eight cysteine residues (e.g., forming four cysteine pairs). Such proteins have a cysteine content of at least 4%. Even more preferably, the microbiota protein (or the fragment or sequence variant thereof) has (i) a length of 20 - 75 amino acids and comprises at least four cysteine residues (e.g., forming two cysteine pairs); (ii) a length of 50 - 100 amino acids and comprises at least six cysteine residues (e.g., forming three cysteine pairs); or (iii) a length of 75 - 125 amino acids and comprises at least eight cysteine residues (e.g., forming four cysteine pairs). Such proteins have a cysteine content of more than 5%.

Exemplified microbiota proteins were identified by the present inventors as described in the Example section. The amino acid sequences of those exemplified microbiota proteins are shown in Table 2 below:

**Table 2: Sequences of exemplified microbiota proteins inducing and/or enhancing IL-10 secretion from human cells.**

| **SEQ ID NO** | **Sequence** |
|---|---|
| SEQ ID NO: 1 | AFLFTSTGVPKKAAEAAFFLYLNKGTKKGSRSCFFIYLDRGTKKG SRSCFFYLPRQGYQKGSRGCFFIYLDRGTKKGSRGCFFIYLDCE KRAGNVCIRKCRGRYLHKKTPRRYRNAEATCS |
| SEQ ID NO: 2 | QTRKQREDAKREAWKKERKEKKALEAQQDSVSFMKDTESCCA SKAFFSLRSFFHASRLASSLCFLVCAFVTPLKQTSNNAAKNNTFFI IKAVLLINISFR |
| SEQ ID NO: 3 | ARNYTCDVCGNGTIQIVSSHIIHNVHCGFIPCNKINGVMDEV VYKTVTENNEACNNCGVSYTYKVYGDMEIICKAKAN |
| SEQ ID NO: 4 | AEPADTAISERRVELCGNCGGRMVTSTTWGSWYTVAQIKCTH HNYGTDLRQQRDGTATTKCQGCGQGYTTSKSQTRIVCHGY DS |
| SEQ ID NO: 5 | LAGPGSGCRFTPSCSTYFIQAVEIHGALKGSLMGIWRILRCNP WGGCGYDPVPPRKPR |
| SEQ ID NO: 6 | LAGPGSGCRFTPSCSTYFIQAVEIHGALKGSLMGIWRILRCNP WGGCGYDPVPPRKPR |
| SEQ ID NO: 7 | AKLGMAAGAMLVLGLLAAGASGGTLILAALALCAVTLLCGRK KQ |
| SEQ ID NO: 8 | VEKKTVITKCAITVNEYREKVVPSMRKIHAIVIFVSYSINHLYKNC EPEQLFSPGRKTKKPPPATCRKRLNLQYF |
| SEQ ID NO: 9 | EITQPCNHVKSDWIIDKEATCIGSYAFYNCTSLTSIEISTSVTKIKY RAFASCRALNNIYYTGTLTQWNEISKDTNWNWAAPLNCKVIC LNGTCYL |
| SEQ ID NO: 10 | LLVSVCTAAGLLAVAMRQIEPLLAWLRTLEVYFQGQSPAVLLR ALGIALVAQFAADTCREAGLCAASTAIELCGRVLVLLQALPLLRS LLGSFADYLQ |

Accordingly, a microbiota protein comprising the sequence as set forth in SEQ ID NO: 1, or a fragment or variant thereof as described above, is preferred. Moreover, a microbiota protein comprising the sequence as set forth in SEQ ID NO: 2, or a fragment or variant thereof as described above, is also preferred. Moreover, a microbiota protein comprising the sequence as set forth in SEQ ID NO: 3, or a fragment or variant thereof as described above, is also preferred. Moreover, a microbiota protein comprising the sequence as set forth in SEQ ID NO: 4, or a fragment or variant thereof as described above, is also preferred. Moreover, a microbiota protein comprising the sequence as set forth in SEQ ID NO: 5, or a fragment or variant thereof as described above, is also preferred. Moreover, a microbiota protein comprising the sequence as set forth in SEQ ID NO: 6, or a fragment or variant thereof as described above, is also preferred. Moreover, a microbiota protein comprising the sequence as set forth in SEQ ID NO: 7, or a fragment or variant thereof as described above, is also preferred. Moreover, a microbiota protein comprising the sequence as set forth in SEQ ID NO: 8, or a fragment or variant thereof as described above, is also preferred. Moreover, a microbiota protein comprising the sequence as set forth in SEQ ID NO: 9, or a fragment or variant thereof as described above, is also preferred. Moreover, a microbiota protein comprising the sequence as set forth in SEQ ID NO: 10, or a fragment or variant thereof as described above, is also preferred. Most preferably, the microbiota protein comprises the sequence as set forth in SEQ ID NO: 1, or a fragment or variant thereof as described above.

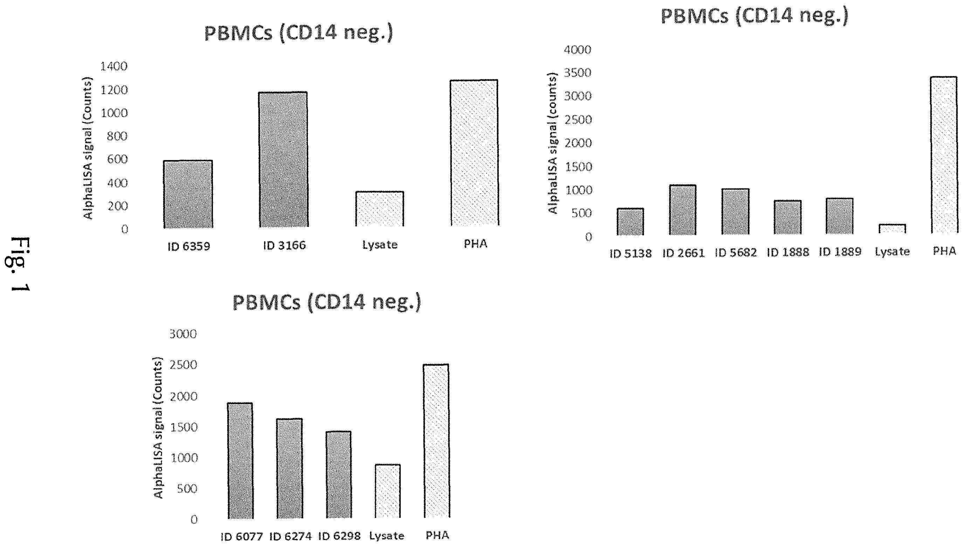

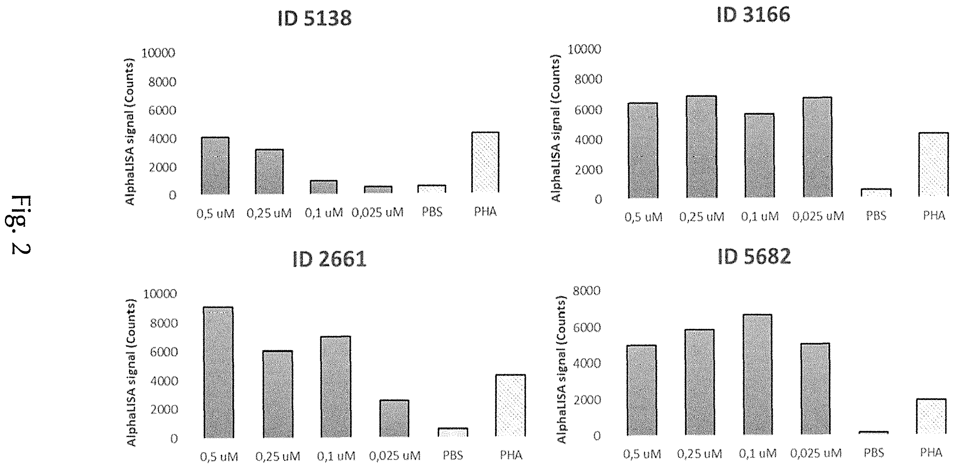

As shown in Example 1 (Fig. 1), IL-10 secretion from human cells stimulated with the exemplified microbiota proteins of SEQ ID NOs 1 - 10 is higher than IL-10 secretion from the same type of human cells stimulated with an *E. coli* lysate. Moreover, as shown in Example 2 (Fig. 2), IL-10 secretion from human cells stimulated with the exemplified microbiota proteins of SEQ ID NOs 1, 5, 6, and 7 is at least as high as IL-10 secretion from the same type of human cells stimulated with phytohemagglutinin (PHA). In particular, IL-10 secretion from human cells stimulated with 0.1 µM of with the exemplified microbiota proteins of SEQ ID NOs 1, 5 and 6 is higher than IL-10 secretion from the same type of human cells stimulated with 10 µg/ml PHA. Moreover, IL-10 secretion from human cells stimulated with concentrations as low as 0.025 µM of the exemplified microbiota proteins of SEQ ID NOs 1 and 6 is still higher than IL-10 secretion from the same type of human cells stimulated with 10 µg/ml PHA.

In some embodiments, the microbiota protein, or the fragment or sequence variant thereof, comprises or consists of an amino acid sequence sharing at least 80% sequence identity with any one of SEQ ID NOs 1 - 10. In particular, the microbiota protein, or the fragment or sequence variant thereof, may comprise or consist of an amino acid sequence sharing at least 80% sequence identity with SEQ ID NO: 1.

### Production of microbiota-derived proteins

The microbiota protein, or the fragment or sequence variant thereof, of the invention may be prepared by recombinant means (i.e., the protein may be prepared artificially). For example, the protein may be prepared by chemical synthesis, *in vitro* or expressed by another organism (other than the organism from which the sequence is obtained; "heterologous expression"). Accordingly, the microbiota protein, or the fragment or sequence variant thereof, of the invention is in particular a recombinant protein. In some embodiments, the microbiota protein, or the fragment or sequence variant thereof, of the invention is prepared by chemical synthesis. In some embodiments, the microbiota protein, or the fragment or sequence variant thereof, of the invention is prepared by *in vitro* synthesis (cell-free expression) or by recombinant overexpression. Preferably, the protein is purified.

The skilled person is aware of various methods to prepare proteins based on the amino acid sequence or the nucleic acid sequence. For example, the protein may be prepared by chemical synthesis, *in vitro* synthesis (cell-free expression) or by (*in vivo*) recombinant (over)expression.

For example, the microbiota protein, or the fragment or sequence variant thereof, of the invention may be expressed heterologously by bacteria, e.g. *E. coli,* transformed with an expression vector comprising a nucleic acid sequence encoding the protein as well-known in the art.

*In vitro* protein synthesis (also referred to as *"in vitro* protein expression", "cell-free protein expression" and "cell-free protein synthesis") is the production of recombinant proteins in solution using biomolecular translation machinery extracted from cells. *In vitro* protein synthesis occurs in cell lysates or in cocktails of recombinant proteins rather than within cultured cells and, thus, it is achieved without the use of living cells. The *in vitro* protein synthesis environment is not constrained by a cell wall or homeostasis conditions necessary to maintain cell viability. Accordingly, *in vitro* protein synthesis enables direct access and control of the translation environment. Moreover, this technique enables rapid expression and manufacture of functional proteins. *In vitro* protein synthesis is useful for various applications including optimization of protein production, optimization of protein complexes, to study protein synthesis, incorporating non-natural amino acids, high-throughput screens, functional analyses, molecular interaction detection, molecular structure and localization analyses and molecular diagnostics.

Common components of a cell-free reaction comprise proteins necessary to achieve *in vitro* transcription and transduction, such as cocktails of recombinant proteins or cell extracts. In addition, an energy source, a supply of amino acids, a nucleic acid encoding the protein to be expressed and, optionally, a cofactor, such as magnesium, may be added. *In vitro* protein synthesis can be accomplished with several kinds and species of cell extracts or with cocktails of recombinant proteins. A cell extract may be obtained, for example, by lysing a cell of interest and centrifuging out the cell walls, DNA genome, and other debris, such that the necessary cell machinery (including ribosomes, aminoacyl-tRNA synthetases, translation initiation and elongation factors, nucleases, etc.) remains. Examples include cell extracts made from *E. coli* (ECE), rabbit reticulocytes (RRL), wheat germ (WGE), insect cells (ICE, for example SF9 or SF21) and human cells, which are all commercially available. Examples of commercially available *in vitro* protein synthesis systems include, but are not limited to, RTS (5 PRIME); Expressway™ (Life Technologies); S30 T7 high yield (Promega); One-step human IVT (Thermo Scientific); WEPRO® (CellFree Sciences); TNT® coupled (Promega); RTS CECF (5 PRIME); TNT® Coupled (Promega); Retic lysate IVT™ (Life Technologies); TNT® T7 (Promega); EasyXpress Insect kit(Qiagen/RiN A); PURExpress® (New England Biolabs); and PURESYSTEM® (BioComber)). In some embodiments, PURExpress® (New England Biolabs) may be used, which is a cocktail of recombinant proteins necessary to achieve in vitro transcription and transduction. In other embodiments, bacterial extracts or lysates, e.g. from *E.* coli, in particular ECE (*E. coli* extract), may be used, since they provide the bacterial machinery (which is ideal for expression of bacterial proteins) and typically achieve high yields. A more detailed description of *in vitro* protein synthesis in bacterial extracts may be derived from Hani S. Zaher, Rachel Green: Chapter One - In Vitro Synthesis of Proteins in Bacterial Extracts, Editor(s): Jon Lorsch, Methods in Enzymology, Academic Press, Vol. 539, 2014, p. 3-15, ISSN 0076-6879, ISBN 9780124201200. Kits for cell-free heterologous expression are commercially available, e.g. *Escherichia coli* Cell Free kit (RTS 100 E. coli Disulfide Kit; Biotechrabbit, Hennigsdorf, Germany).

The nucleic acid used in *in vitro* protein synthesis is preferably RNA or DNA. For example, isolated RNA (in particular mRNA) synthesized *in vivo* or *in vitro* may be used as template for translation. It is also preferred to use DNA, in particular a coupled translation/transcription system, in which circular or linear DNA, such as a gene/an ORF cloned into a plasmid vector (cDNA), or a linear DNA template, such as a PCR-generated template, are used. The nucleic acid may be codon optimized. In some embodiments, direct synthesis of DNA is used, preferably with codon optimization.

The nucleic acid, e.g., a synthetic DNA molecule, may be subcloned into a vector. The vector may be an expression vector, which may be used for production of expression products such as peptides, polypeptides or proteins. For example, an expression vector may comprise sequences needed for transcription of a sequence stretch of the vector, such as a promoter sequence (e.g., a T7 promoter). Accordingly, the vector may comprise a (T7) promoter. In addition, the vector may contain a particular tag, if required. In some embodiments, the vector contains control elements for heterologous expression of the microbiota protein, or the fragment or sequence variant thereof, of the invention in bacterial cells.

*In vitro*/cell-free protein synthesis is preferably applied to proteins having a minimum length of 20 amino acids, preferably having a minimum length of 30 amino acids, more preferably having a minimum length of 40 amino acids, even more preferably having a minimum length of 45 amino acids and still more preferably having a minimum length of 50 amino acids. For example, proteins having a length (preferably without signal peptide) of 50 to 350 amino acids or 50 to 500 amino acids are synthesized by *in vitro*/cell-free protein synthesis as described above.

Alternatively, the microbiota protein, or the fragment or sequence variant thereof, of the invention may be prepared by chemical synthesis, as known in the art and described, for example, by Fields GB. Introduction to peptide synthesis. Curr Protoc Protein Sci. 2002; Chapter 18:Unit-18.1. doi:10.1002/0471140864.ps1801s26. For example, solid phase technologies known in the art (solid-phase peptide synthesis (SPPS)) may be used, e.g. applying Fmoc-based chemistries. SPPS allows the rapid assembly of a peptide chain through successive reactions of amino acid derivatives on an insoluble porous support. Various commercial suppliers of chemical protein and peptide synthesis are available, e.g. Pepscan (Lelystad, Netherlands), GenScript (Piscataway, NJ, USA), LifeTein (Somerset, NJ, USA), JPT (Berlin, Germany), Smart Bioscience (Saint Egrève, France).

Chemical synthesis, e.g. SPPS, may be applied to proteins having a maximum length of 100 amino acids, preferably having a maximum length of 90 amino acids, more preferably having a maximum length of 80 amino acids, even more preferably having a maximum length of 70 or 60 amino acids and still more preferably having a maximum length of 50 amino acids. For example, proteins having a length (preferably without signal peptide) of 15 to 50 amino acids or 20 to 50 amino acids are synthesized by chemical synthesis, e.g. SPPS.

Accordingly, longer proteins, e.g. having a length as described above (e.g., 50 - 350 amino acids), may be synthesized by *in vitro*/cell*-*free protein synthesis and shorter proteins, e.g. having a length as described above (e.g., 20 - 50 amino acids) may be synthesized by chemical synthesis, e.g. SPPS.

### Nucleic acids and vectors encoding the microbiota-derivedprotein

In a further aspect, the present invention also provides a nucleic acid comprising a polynucleotide encoding the microbiota protein, or the fragment or sequence variant thereof, according to the present invention as described above.

Nucleic acids preferably comprise single stranded, double stranded or partially double stranded nucleic acids, preferably selected from genomic DNA, cDNA, RNA, siRNA, antisense DNA, antisense RNA, ribozyme, complimentary RNA/DNA sequences with or without expression elements, a mini-gene, gene fragments, regulatory elements, promoters, and combinations thereof. Further preferred examples of nucleic acid (molecules) include, e.g., a recombinant polynucleotide, a vector, an oligonucleotide, an RNA molecule such as an rRNA, an mRNA or a tRNA, or a DNA molecule as described above. It is thus preferred that the nucleic acid (molecule) is a DNA molecule or an RNA molecule; preferably selected from genomic DNA; cDNA; rRNA; mRNA; antisense DNA; antisense RNA; complimentary RNA and/or DNA sequences; RNA and/or DNA sequences with or without expression elements, regulatory elements, and/or promoters; a vector; and combinations thereof.

Nucleic acids encoding proteins according to the invention may be in the form of naked nucleic acids, or nucleic acids cloned into plasmids or viral vectors (Tregoning and Kinnear, Using Plasmids as DNA Vaccines for Infectious Diseases. Microbiol Spectr. 2014 Dec;2(6). doi: 10.1128/microbiolspec.PLAS-0028-2014), the latter being particularly preferred. Examples of suitable viral vectors according to the invention include, without limitation, retrovirus, adenovirus, adeno-associated virus (AAV), herpes virus and poxvirus vectors. It is within the skill of the person in the art to clone a nucleic acid into a plasmid or viral vector, using standard recombinant techniques in the art.

In particular, preferred embodiments of the microbiota protein, or the fragment or sequence variant thereof, according to the present invention as described above also apply for such a nucleic acid according to the present invention. For example, the nucleic acid preferably encodes the microbiota protein comprising or consisting of an amino acid sequence as set forth in any one of SEQ ID NOs 1 to 10, or a fragment or sequence variant thereof, as described above. Exemplified nucleic acid sequences encoding the microbiota proteins as set forth in SEQ ID NO 1 - 10 are provided in Table 3 below:

**Table 3: Exemplified nucleic acid sequences encoding the microbiota proteins as set forth in SEQ ID NO 1 - 10.**

| **Nucleic acid SEQ ID NO** | **Nucleic acid sequence** | **Encoded protein SEQ ID NO** |
|---|---|---|
| SEQ ID NO: 11 | GCTTTTTTATTTACCTCGACAGGGGTACCAAAAA AGGCAGCAGAGGCTGCTTTTTTTCTTTACCTCAA CAAGGGTACCAAAAAAGGCAGCAGAAGCTGCT TTTTTATTTACCTCGACAGGGGTACCAAAAAAGG CAGCAGAAGCTGCTTTTTTTATTTACCTCGACAG GGGTACCAAAAAGGCAGCAGAGGCTGCTTTTTT ATTTACCTCGACAGGGGTACCAAAAAAGGTAGC AGAGGCTGCTTTTTTATTTACCTCGATTGCGAAA AGCGCGCAGGCAATGTCTGCATACGCAAATGCC GCGGGCGGTATTTGCACAAAAAAACGCCGCGG CGGTATCGAAACGCCGAGGCGACGTGCTCCTA A | SEQ ID NO: 1 |
| SEQ ID NO: 12 | CAAACCAGAAAACAAAGAGAAGATGCCAAACG CGAAGCATGGAAAAAAGAACGTAAGGAAAAGA AGGCCTTGGAAGCACAGCAAGATTCCGTGTCTT TCATGAAAGACACGGAATCTTGCTGTGCTTCCAA GGCCTTCTTTTCCTTACGTTCTTTTTTCCATGCTTC GCGTTTGGCATCTTCTCTTTGTTTTCTGGTTTGTG CATTTGTTACTCCGCTGAAGCAAACAAGTAACAA TGCTGCTAAGAATAATACTTTTTTCATAATTAAAG CTGTTTTGTTAATCAATATATCTTTTAGATAA | SEQ ID NO: 2 |
| SEQ ID NO: 13 | GCAAGGAACTATACATGTGATGTATGTGGAAAT GGAACAATACAAATCGTTTCTTCTCATATAATCCA TAATGTACACTGTGGATTCATTCCATGTAATAAA ATTAACGGAGTTATGGACGAAGTTGTTTATAAAA CTGTTACAGAAAATAACGAAGCTTGTAACAACTG TGGTGTTTCTTATACTTATAAAGTATATGGGGAC ATGGAAATAATATGTAAAGCTAAAGCAAATTAG | SEQ ID NO: 3 |
| SEQ ID NO: 14 | GCCGAACCGGCGGACACCGCGATTTCTGAACG GCGCGTGGAACTTTGCGGCAACTGCGGCGGAC GAATGGTAACTTCCACTACATGGGGAAGCTGGT ACACAGTTGCTCAAATCAAATGCACACACCATAA TTATGGTACCGATCTCCGTCAGCAGAGAGACGG TACGGCAACAACAAAGTGCCAGGGCTGCGGAC AGGGTTACACCACTTCCAAATCTCAGACAAGAAT TGTTTGCCACGGTTACGATTCCTAA | SEQ ID NO: 4 |
| SEQ ID NO: 15 | GCAGCATTTGTTTTTTCCAACTCGCTCAAACCCG CTAATGCCTCAAGCGCGGAAAGCAGCCGGCTC CTCATCCATGTGAACTCATTTTTTTCACAGCTTGG GCTTAAACCGATAAGCGAAAACCTGCTTCGCAA AACGGCGCATTTCTGCGAGTTCGGTATGCTCGG AATACTTGCATCCTCCGCCTGCGCAATGTTTTCC GGAGCTTATTCTGCCGCCTCCCTGCCCTCTCTGC GCCGCCGTGGCTTTTTCATCTCCTTTGGCGTATC TGTCGCGTGCGCCGTATGTGACGAAACGATCCA GTATTTTGTTCCCGGCAGAGCCTGCCGCGTGAC CGATATGCTCATCGACTCCGCCGGAGCACTCTG CGGGCTTGCTGCCGTACTTGCGTTCTGCGCGGC AATTCGCGTCCGGCGCCGGCGCCGCCGGAATT GA | SEQ ID NO: 5 |
| SEQ ID NO: 16 | CTGGCCGGGCCGGGTTCCGGCTGTCGGTTCAC CCCCAGCTGTTCGACCTACTTCATTCAGGCCGTG GAGATTCACGGCGCGCTCAAGGGCTCGCTGAT GGGCATCTGGCGCATTCTGCGCTGCAATCCTTG GGGCGGCTGCGGCTATGATCCCGTGCCGCCGC GAAAGCCCCGATGA | SEQ ID NO: 6 |
| SEQ ID NO: 17 | GCAAAGCTCGGCATGGCGGCGGCGCAATGCT GGTGCTCGGGCTGCTGGCGGCGGGTGCCTCCG GCGGCACGCTCATCCTTGCGGCGCTGGCACTGT GTGCCGTGACCCTGTTGTGCGGGAGGAAAAAG CAATGA | SEQ ID NO: 7 |
| SEQ ID NO: 18 | GTGGAAAAGAAAACAGTGATTACGAAATGTGCA ATCACAGTAAACGAATACCGCGAAAAAGTTGTC CCGTCAATGCGCAAAATTCACGCCATTGTCATAT TTGTGAGCTACTCAATAAATCATTTGTACAAAAAT TGTGAGCCAGAACAGCTATTTTCACCGGGCAGA AAAACAAAAAAACCGCCCCCGGCGACGTGCCG AAAGCGGTTAAATTTACAATATTTTTAA | SEQ ID NO: 8 |
| SEQ ID NO: 19 | GAAATAACACAACCATGTAATCATGTTAAAAGTG ATTGGATAATAGATAAAGAAGCAACATGTATAG GAAGTTATGCCTTTTACAATTGTACTAGTTTAACA AGTATAGAAATTTCAACAAGTGTTACAAAAATAA AATATCGTGCATTTGCTAGTTGTCGAGCTTTAAA TAATATATACTACACAGGAACACTAACTCAATGG AATGAAATAAGTAAGGATACTAATTGGAATTGG GCTGCACCATTAAATTGTAAAGTTATTTGTTTAAA TGGTACATGTTACTTATAA | SEQ ID NO: 9 |
| SEQ ID NO: 20 | CTTTTGGTCTCGGTATGCACCGCCGCCGGGCTG CTTGCTGTGGCGATGCGCCAGATCGAGCCGCT GCTCGCTTGGCTGCGCACACTGGAGGTCTACTT TCAGGGGCAGAGCCCGGCGGTGCTGCTGCGC GCTCTCGGCATTGCGCTGGTCGCGCAGTTTGCC GCCGACACCTGCCGCGAGGCCGGTCTTTGCGC GGCCTCCACCGCCATCGAGCTCTGCGGCCGTGT GCTGGTGCTTTTGCAGGCTCTGCCGCTGCTGCG GTCGCTGCTCGGCTCCTTTGCGGATTATTTGCAG TAA | SEQ ID NO: 10 |

Accordingly, it is preferred that the polynucleotide encoding the microbiota protein, or the fragment or sequence variant thereof, has a nucleic acid sequence as set forth in any one of SEQ ID NOs 11 - 20 or sequence variants thereof as described above. For example, the nucleic acid sequence of said polynucleotide may share at least 80% sequence identity with any one of SEQ ID NOs 11 - 20. More preferably, the polynucleotide encoding the microbiota protein, or the fragment or sequence variant thereof, has a nucleic acid sequence according to any one of SEQ ID NOs 11 - 20. However, it is understood that due to the redundancy of the genetic code, distinct nucleic acid sequences may encode the same amino acid sequence. Accordingly, the proteins of SEQ ID NOs 1 - 10 can also be encoded by nucleic acids distinct from the above-mentioned exemplified nucleic acids.

Due to the redundancy of the genetic code, the present invention also comprises sequence variants of nucleic acid sequences, which encode the same amino acid sequences. The polynucleotide encoding the microbiota protein, or the fragment or sequence variant thereof, may be optimized for expression of the microbiota protein, or the fragment or sequence variant thereof. For example, codon optimization of the nucleotide sequence may be used to improve the efficiency of translation in expression systems for the production of the microbiota protein, or the fragment or sequence variant thereof. Moreover, the nucleic acid molecule may comprise heterologous elements (i.e., elements, which in nature do not occur on the same nucleic acid molecule as the coding sequence for the microbiota protein, or the fragment or sequence variant thereof. For example, a nucleic acid molecule may comprise a heterologous promotor, a heterologous enhancer, a heterologous UTR (e.g., for optimal translation/expression), a heterologous Poly-A-tail, and the like.

A nucleic acid (molecule) is a molecule comprising nucleic acid components. The term nucleic acid (molecule) usually refers to DNA or RNA (molecules). It may be used synonymous with the term "polynucleotide", i.e. the nucleic acid molecule may consist of a polynucleotide encoding the microbiota protein, or the fragment or sequence variant thereof. Alternatively, the nucleic acid molecule may also comprise further elements in addition to the polynucleotide encoding the microbiota protein, or the fragment or sequence variant thereof. Typically, a nucleic acid molecule is a polymer comprising or consisting of nucleotide monomers which are covalently linked to each other by phosphodiester-bonds of a sugar/phosphate-backbone. The term "nucleic acid (molecule)" also encompasses modified nucleic acid (molecules), such as base-modified, sugar-modified or backbone-modified etc. DNA or RNA molecules.

In general, the nucleic acid (molecule) may be manipulated to insert, delete or alter certain nucleic acid sequences. Changes from such manipulation include, but are not limited to, changes to introduce restriction sites, to amend codon usage, to add or optimize transcription and/or translation regulatory sequences, etc. It is also possible to change the nucleic acid to alter the encoded amino acids. For example, it may be useful to introduce one or more (*e.g.,* 1, 2, 3, 4, 5, 6, 7, 8, 9, 10, etc.) amino acid substitutions, deletions and/or insertions into the human microbiota protein's amino acid sequence. Such point mutations can modify effector functions, post-translational modifications, immunogenicity, etc., can introduce amino acids for the attachment of covalent groups (e.g., labels) or can introduce tags (e.g., for purification purposes). Alternatively, a mutation in a nucleic acid sequence may be "silent", i.e. not reflected in the amino acid sequence due to the redundancy of the genetic code. In general, mutations can be introduced in specific sites or can be introduced at random, followed by selection (e.g., molecular evolution). For instance, one or more nucleic acids encoding any of the microbiota protein, or the fragment or sequence variant thereof, of the invention can be randomly or directionally mutated to introduce different properties in the encoded amino acids. Such changes can be the result of an iterative process wherein initial changes are retained and new changes at other nucleotide positions are introduced. Further, changes achieved in independent steps may be combined.

In some embodiments, the polynucleotide encoding the microbiota protein, or the fragment or sequence variant thereof, (or the (complete) nucleic acid molecule) may be codon-optimized. The skilled artisan is aware of various tools for codon optimization, such as those described in: Ju Xin Chin, Bevan Kai-Sheng Chung, Dong-Yup Lee, Codon Optimization OnLine (COOL): a web-based multi-objective optimization platform for synthetic gene design, Bioinformatics, Volume 30, Issue 15, 1 August 2014, Pages 2210-2212; or in: Grote A, Hiller K, Scheer M, Munch R, Nortemann B, Hempel DC, Jahn D, JCat: a novel tool to adapt codon usage of a target gene to its potential expression host. Nucleic Acids Res. 2005 Jul 1;33(Web Server issue):W526-31; or in US 2011/0081708 A1; or as provided by commercial suppliers, e.g., the codon optimization algorithm provided by Twist Bioscience (San Francisco, USA). In some embodiments, the polynucleotide encoding the microbiota protein, or the fragment or sequence variant thereof, (or the (complete) nucleic acid molecule) is codon-optimized for expression by prokaryotic cells, preferably it is codon-optimized for expression in bacteria, such as *E. coli.*

In a further aspect, the present invention also provides an expression cassette comprising the polynucleotide encoding the microbiota protein, or the fragment or sequence variant thereof, of the invention; or the nucleic acid (molecule) of the invention as described above. In addition, an expression cassette usually comprises - operably linked to said polynucleotide or nucleic acid encoding the microbiota protein, or the fragment or sequence variant thereof - a regulatory element for expression of the microbiota protein, or the fragment or sequence variant thereof.

The regulatory element may be a (heterologous) promoter. The promoter initiates the transcription and is therefore the point of control for the expression of the encoded protein. The promoter may be inducible, such that protein synthesis is only initiated when required by the introduction of an inducer, such as IPTG. However, gene expression however may also be constitutive (i.e. the protein may be constantly expressed). Examples of promoters include the promoter of the *lac* operon or the T7 promoter. They may be regulated by the *lac* operator. A promoter may also be a hybrid of different promoters, for example, the Tac-Promoter, which is a hybrid of *trp* and *lac* promoters.

For example, the expression cassette may comprise (operably linked to each other): a (heterologous) promoter, an open reading frame (ORF) comprising or consisting of the polynucleotide encoding the microbiota protein, or the fragment or sequence variant thereof, of the invention, and a 3' untranslated region (3'UTR). In some embodiments, the expression cassette comprises (operably linked to each other): a (heterologous) promoter, a ribosomal binding site, the polynucleotide encoding the microbiota protein, or the fragment or sequence variant thereof, of the invention, and a transcription terminator (for terminating DNA transcription).

An expression cassette typically comprises the gene to be expressed (in the present case the polynucleotide/nucleic acid encoding the microbiota protein, or the fragment or sequence variant thereof) and a regulatory element for expression. An expression cassette may be a component of a vector, in particular of an expression vector, such that the encoded protein can be expressed by a cell comprising said vector, e.g. a cell transfected with said vector. The expression cassette usually directs the cell's machinery to produce RNA and protein(s). In some embodiments, the expression cassette may be designed for modular cloning of protein-encoding sequences, such that the same cassette can easily be altered to make different proteins. The regulatory element of the expression cassette is usually heterologous in comparison to the encoded protein (to be expressed), i.e. the specific combination of regulatory element and polynucleotide sequence encoding the protein of interest does typically not occur in nature. The regulatory element usually controls the expression of the encoded protein.

The expression cassette may further comprise a nucleic acid sequence encoding a tag (for example, directly downstream of the polynucleotide encoding the microbiota protein, or the fragment or sequence variant thereof, according to the present invention), such that the expressed protein contains a tag. A tag may be useful, for example, for purification of the protein after expression or as reporters (labels). Examples of tags include histidine (His) tags, other marker peptides, or fusion partners such as glutathione S-transferase or maltose-binding protein.

The expression cassette may be optimized for expression in eukaryotic or prokaryotic cells. For example, an expression cassette for prokaryotes expression vectors may include a Shine-Dalgarno sequence at its translation initiation site for the binding of ribosomes, while an expression cassette for eukaryotic expression may contain the Kozak consensus sequence.

Preferably, the expression cassette is optimized for expression in a prokaryotic cell, such as a bacterium.

Further included within the scope of the invention are vectors, for example, expression vectors, comprising the nucleic acid according to the present invention as described above or the expression cassette according to the present invention as described above. For example, the nucleic acid molecule as described above may be a vector. Accordingly, the present invention also provides a vector, comprising the nucleic acid according to the present invention as described above or the expression cassette according to the present invention as described above.

A vector is usually a (recombinant) nucleic acid molecule, which does not occur in nature. Accordingly, the vector may comprise heterologous elements (i.e., sequence elements of different origin in nature). For example, the vector may comprise a multi cloning site, a heterologous promotor, a heterologous enhancer, a heterologous selection marker (to identify cells comprising said vector in comparison to cells not comprising said vector) or combinations thereof. A vector in the context of the present invention is suitable for incorporating or harboring a desired nucleic acid sequence. Such vectors may be storage vectors, expression vectors, cloning vectors, transfer vectors etc. A storage vector is a vector which allows the convenient storage of a nucleic acid molecule. Thus, the vector may comprise a sequence corresponding to (or encoding), e.g., the microbiota protein, or the fragment or sequence variant thereof, according to the present invention. An expression vector may be used for production of expression products such as RNA, e.g. mRNA, or peptides, polypeptides or proteins. For example, an expression vector may comprise sequences needed for transcription of a sequence stretch of the vector, such as a (heterologous) promoter sequence, e.g. as described above. A cloning vector is typically a vector that contains a cloning site, which may be used to incorporate nucleic acid sequences into the vector. A cloning vector may be, e.g., a plasmid vector or a bacteriophage vector. A transfer vector may be a vector which is suitable for transferring nucleic acid molecules into cells or organisms, for example, viral vectors.

A vector in the context of the present invention may be, e.g., an RNA vector or a DNA vector. Preferably, a vector is a DNA molecule. For example, a vector in the sense of the present application comprises a cloning site, a selection marker, such as an antibiotic resistance factor, and a sequence suitable for multiplication of the vector, such as an origin of replication. Preferably, a vector in the context of the present application is a plasmid vector. Preferably, a vector in the context of the present application is an expression vector. An expression vector may further comprise a nucleic acid sequence encoding a tag (for example, directly downstream of the polynucleotide encoding the microbiota protein, or the fragment or sequence variant thereof, according to the present invention), such that the expressed protein contains a tag. A tag may be useful, for example, for purification of the protein after expression or as reporters (labels). Examples of tags include histidine (His) tags, other marker peptides, or fusion partners such as glutathione S-transferase or maltose-binding protein.

A preferred vector is a vector for expression in bacterial cells, such as *E. coli.* Many expression vectors for expression of a protein of interest in bacterial cells, such as *E. coli,* are commercially available.

The vector may be useful for expression in so-called "live bacterial vectors", wherein live bacterial cells (such as bacteria or bacterial spores, e.g., endospores, exospores or microbial cysts) can be administered. Preferred examples thereof are described in Pálffy R, Gardlík R, Hodosy J, Behuliak M, Resko P, Radvánský J, Celec P. Bacteria in gene therapy: bactofection versus alternative gene therapy. Gene Ther. 2006 Jan;13(2):101-5.

### Cells and culture medium

In a further aspect, the present invention also provides a (host) cell expressing the microbiota protein, or the fragment or sequence variant thereof, according to the invention; or comprising the nucleic acid, the expression cassette or the vector according to the present invention. The (host) cell may be an isolated cell.

Examples of such cells include but are not limited to, eukaryotic cells, e.g., yeast cells, animal cells or plant cells or prokaryotic cells, including *E. coli.* In some embodiments, the cells are mammalian cells, such as a mammalian cell line. Examples include human cells, CHO cells, HEK293T cells, PER.C6 cells, NS0 cells, human liver cells, myeloma cells or hybridoma cells.

Preferably, the (host) cell is a bacterial cell, such as an *E. coli* cell. Such a cell is preferably used for production of the microbiota protein, or the fragment or sequence variant thereof, according to the present invention. Moreover, such the (host) cell may also be an active component in a pharmaceutical composition.

Preferably, the (host) cell is a bacterial cell, more preferably a gut bacterial cell. Such a bacterial cell may serve as "live bacterial vector", wherein live bacterial cells (such as bacteria or bacterial spores, e.g., endospores, exospores or microbial cysts) can serve as vectors to provide the protein of the invention or a nucleic acid encoding the same. Preferred examples thereof are described in Pálffy R, Gardlík R, Hodosy J, Behuliak M, Resko P, Radvánský J, Celec P. Bacteria in gene therapy: bactofection versus alternative gene therapy. Gene Ther. 2006 Jan;13(2):101-5. Bacterial cells (such as bacteria or bacterial spores, e.g., endospores, exospores or microbial cysts), in particular (entire) gut bacterial species, can be advantageous, as they have the potential to trigger a greater immune response than the proteins or nucleic acids they contain.

Moreover, bacterial cells, in particular gut bacteria, according to the invention may be provided in the form of probiotics, i.e. of live gut bacterium, which can thus be used as food additive due to the health benefits it can provide. Those can be for example lyophilized in granules, pills or capsules, or directly mixed with dairy products for consumption.

Accordingly, the present invention also provides a genetically engineered bacterium capable of inducing and/or enhancing IL-10 secretion from a human cell, the bacterium comprising the nucleic acid according to the present invention, the expression cassette according to the present invention, or the vector according to the present invention as described above. Furthermore, the present invention also provides a genetically engineered bacterium (over)expressing the protein, or the fragment or sequence variant thereof, according to the present invention.

Preferably, the bacterial (host) cell/bacterium is an engineered bacterium. For example, a bacterial (host) cell may be genetically modified for better penetration through the cellular membrane; for the facilitation of the release of the carried plasmid or expressed protein; or to reduce the risk of clinically symptomatic infections to a minimum. Engineered bacteria are described, for example, in Pálffy R, Gardlík R, Hodosy J, Behuliak M, Resko P, Radvánský J, Celec P. Bacteria in gene therapy: bactofection versus alternative gene therapy. Gene Ther. 2006 Jan;13(2):101-5. Engineered bacteria secreting proteins of interest are described, for example, in WO 2016/164636 or in WO 2018/045184.

In general, the (engineered) bacterium is preferably a non-pathogenic bacterium. The term "non-pathogenic bacterium", as used herein, refers to bacteria that are essentially not capable of causing disease or harmful responses in a host. In some embodiments, non-pathogenic bacteria are commensal bacteria. Examples of non-pathogenic bacteria include, but are not limited to *Bacillus, Bacteroides, Bifidobacterium, Brevibacteria, Clostridium, Enterococcus, Escherichia coli* (*E. coli*), *Lactobacillus, Lactococcus, Saccharomyces,* and *Staphylococcus,* e.g., *Bacillus coagulans, Bacillus subtilis, Bacteroides fragilis, Bacteroides subtilis, Bacteroides thetaiotaomicron, Bifidobacterium bifidum, Bifidobacterium infantis, Bifidobacterium lactis, Bifidobacterium longum, Clostridium butyricum, Enterococcus faecium, Lactobacillus acidophilus, Lactobacillus bulgaricus, Lactobacillus casei, Lactobacillus johnsonii, Lactobacillus paracasei, Lactobacillus plantarum, Lactobacillus reuteri, Lactobacillus rhamnosus, Lactococcus lactis,* and *Saccharomyces boulardii.* Among those bacteria, (engineered) *E. coli* is particularly preferred.

In a further aspect, the present invention also provides a culture medium comprising the microbiota protein, or the fragment or sequence variant thereof, according to the invention, the (host) cell according to the invention, or the bacterium according to the invention.

The culture medium is preferably a culture medium for human cells, in particular human immune cells. Various culture media for human immune cells, such as PBMCs, are known in the art and commercially available. Examples include RPMI-1640 medium, Iscove's Modified Dulbecco's Medium (IMDM), TexMACS medium, and AIM V Medium (ThermoFisher). The culture medium may be supplemented, e.g. with antibiotics, glutamine and/or (inactivated) fetal bovine serum (FBS). In some embodiments, the culture medium is serum-free. Preferably, the culture medium does not contain IL-10 and/or compounds inducing IL-10 secretion from human cells other than the microbiota protein, or the fragment or sequence variant thereof, according to the invention, the (host) cell according to the invention, or the bacterium according to the invention.

In some embodiments, the culture medium further comprises an antigen, e.g. a self antigen. For instance, in allergy and autoimmunity, patient-derived immune cells (such as dendritic cells) pulsed with a specific antigen (Ag) can be used to induce differentiation of autologous Ag-specific T regulatory type 1 (Tr1) cell products and, therefore, promote/restore Ag-specific tolerance.

In a further aspect, the present invention also provides an isolated human cell cultured with the culture medium as described above, i.e. comprising the microbiota protein, or the fragment or sequence variant thereof, according to the invention, the (host) cell according to the invention, or the bacterium according to the invention. The microbiota protein, or the fragment or sequence variant thereof, according to the invention (or cells expressing such proteins) are capable of inducing and/or enhancing IL-10 secretion from human cells as described above. Preferably, the human cell cultured with the microbiota protein, or the fragment or sequence variant thereof, (or cells expressing such proteins) is a human immune cell, e.g. a peripheral blood mononuclear cell (PBMC). For example, various blood cell types, including lymphocytes, monocytes, dendritic cells and granulocytes were reported to produce IL-10. In some embodiments, the human cell cultured with the microbiota protein, or the fragment or sequence variant thereof, (or cells expressing such proteins) is a lymphocyte, e.g. a T lymphocyte.

As described above, culturing human cells (as described above) in the presence of the microbiota protein, or the fragment or sequence variant thereof, (or cells expressing such proteins) results in inducing and/or enhancing IL-10 release from the human cells. Further details regarding Il-10 secretion of human cells in the presence of the microbiota protein, or the fragment or sequence variant thereof, are provided above, which apply here accordingly.

### Pharmaceutical compositions, medical treatment and uses

In a further aspect, the present invention provides a pharmaceutical composition comprising
- the microbiota protein, or the fragment or sequence variant thereof, as described above;
- the nucleic acid as described above;
- the vector as described above;
- the (host) cell as described above;
- the bacterium as described above; or
- the human cell as described above.

Preferably, the pharmaceutical composition further comprises a pharmaceutically acceptable excipient, diluent or carrier. Although the carrier or excipient may facilitate administration, it should not itself induce the production of antibodies harmful to the individual receiving the composition. Nor should it be toxic. Suitable carriers may be large, slowly metabolized macromolecules such as proteins, polypeptides, liposomes, polysaccharides, polylactic acids, polyglycolic acids, polymeric amino acids, amino acid copolymers and inactive virus particles. In some embodiments, the pharmaceutically acceptable carrier, diluent and/or excipient in the pharmaceutical composition according to the present invention is not an active component in respect to inducing and/or enhancing IL-10 secretion from human cells.

Pharmaceutically acceptable salts can be used, for example mineral acid salts, such as hydrochlorides, hydrobromides, phosphates and sulphates, or salts of organic acids, such as acetates, propionates, malonates and benzoates.

Pharmaceutically acceptable carriers in a pharmaceutical composition may additionally contain liquids such as water, saline, glycerol and ethanol. Additionally, auxiliary substances, such as wetting or emulsifying agents or pH buffering substances, may be present in such compositions. Such carriers enable the pharmaceutical compositions to be formulated as tablets, pills, dragees, capsules, liquids, gels, syrups, slurries and suspensions, for ingestion by the subject. A thorough discussion of pharmaceutically acceptable carriers is available in Gennaro (2000) Remington: The Science and Practice of Pharmacy, 20th edition, ISBN: 0683306472.

Pharmaceutical compositions of the invention may have a pH between 5.5 and 8.5, in some embodiments this may be between 6 and 8, for example about 7. The pH may be maintained by the use of a buffer. The composition may be sterile and/or pyrogen free. The composition may be isotonic with respect to humans. In some embodiments pharmaceutical compositions of the invention are supplied in hermetically-sealed containers.

In some embodiments, the (only) active ingredient in the composition is the microbiota protein, or the fragment or sequence variant thereof, the nucleic acid, the vector, the (host) cell, the bacterium or the human cell according to the present invention as described above. As such, it may be susceptible to degradation in the gastrointestinal tract. Thus, if the composition is to be administered by a route using the gastrointestinal tract, the composition may contain agents which protect the said active component from degradation.

In some embodiments, the microbiota protein, or the fragment or sequence variant thereof, may be administered in the form of a micro-organism such as a (gut) bacterial cell. Moreover, (gut) bacteria according to the invention may be in the form of probiotics, i.e. of a live bacterium, which can thus be used as food additive due to the health benefits it can provide. Those can be for example lyophilized in granules, pills or capsules, or directly mixed with dairy products for consumption.

The composition according to the invention can further comprise other active agents, for example such, which can enhance the effects of the microbiota(-derived) protein of the invention. Alternatively, the composition may not comprise any other active agents (i.e., other than the microbiota(-derived) protein according to the present invention, the cell according to the present invention, the nucleic acid according to the present invention, or the host cell according to the present invention.

In view of the IL-10 secretion induced and/or enhanced by the microbiota protein, or the fragment or sequence variant thereof, according to the present invention, the present invention also provides a method for inducing and/or enhancing IL-10 secretion in a subject, comprising the step of administering to said subject the microbiota protein, or the fragment or sequence variant thereof, according to the present invention, the nucleic acid according to the present invention, the vector according to the present invention, the (host) cell according to the present invention, the bacterium according to the present invention.

Preferably, the microbiota protein, or the fragment or sequence variant thereof, according to the present invention, the nucleic acid according to the present invention, the vector according to the present invention, the (host) cell according to the present invention, the bacterium according to the present invention, or the human immune cell according to the present invention may be used as a medicament.

In other words, the present invention also provides the microbiota protein, or the fragment or sequence variant thereof, according to the present invention, the nucleic acid according to the present invention, the vector according to the present invention, the (host) cell according to the present invention, the bacterium according to the present invention, the human immune cell according to the present invention, or the pharmaceutical composition according to the present invention for use in medicine.

In particular, the microbiota protein, or the fragment or sequence variant thereof, according to the present invention, the nucleic acid according to the present invention, the vector according to the present invention, the (host) cell according to the present invention, the bacterium according to the present invention, the human immune cell according to the invention, or the pharmaceutical composition according to the present invention may be useful in various medical applications including treatment of inflammatory diseases, autoimmune disorders, and diseases of the gastrointestinal tract (GIT), such as inflammatory bowel diseases (IBD). Inflammatory bowel diseases (IBD) include, for example, Crohn's disease and ulcerative colitis.

Accordingly, the present invention provides a method for reducing, treating, alleviating symptoms of or ameliorating an inflammatory disease or an autoimmune disorder in a subject, comprising the step of administering to said subject the microbiota protein, or the fragment or sequence variant thereof, according to the present invention, the nucleic acid according to the present invention, the vector according to the present invention, the (host) cell according to the present invention, the bacterium according to the present invention, the human immune cell according to the present invention or the pharmaceutical composition according to the present invention.

IL-10 is often considered as the "prototype of anti-inflammatory cytokines" and its inhibitory action is exerted mainly against the most typical markers of inflammation such as IL-1, IL-6, TNF-α, GM-CSF and IFN-γ. The three major pro-inflammatory cytokines IL-1β, IL-6, and TNF-α are responsible for the onset and the maintenance of the inflammatory state in many diseases. Moreover, a TH1 immune response is known to be a characteristic of autoimmune and inflammatory diseases. While IL-10 is able to inhibit both the Th1-type and the Th2-type responses, the effect on Th1 subpopulation is predominant and IL-10 is considered as a promoter of the Th2 response (pleiotropic effect). Accordingly, inhibition/reduction of pro-inflammatory cytokines and Th1 immune responses by inducing or enhancing secretion of IL-10 from human cells is helpful in various inflammatory and autoimmune diseases.

Inflammatory diseases include a vast array of disorders and conditions that are characterized by inflammation. Inflammatory diseases may be acute or chronic. Examples of inflammatory diseases include allergy, asthma, chronic obstructive pulmonary disease (COPD), inflammatory bowel disease such as Crohn's disease and ulcerative colitis, psoriasis, rheumatoid arthritis, systemic lupus erythematosus (SLE) and Type 1 diabetes (T1D).

Accordingly, the present invention also provides a method for inducing tolerance in a subject, comprising the step of administering to said subject the microbiota protein, or the fragment or sequence variant thereof, according to the present invention, the nucleic acid according to the present invention, the vector according to the present invention, the (host) cell according to the present invention, the bacterium according to the present invention, or the human immune cell according to the present invention.

### BRIEF DESCRIPTION OF THE FIGURES

In the following a brief description of the appended figures will be given. The figures are intended to illustrate the present invention in more detail. However, they are not intended to limit the subject matter of the invention in any way.
- Figure 1: shows for Example 1 the results of AlphaLISA for IL-10 secretion from human PBMCs by stimulation with the 10 exemplified microbiota proteins in the second round of cell-free synthesis.
- Figure 2:: shows for Example 2 the results of AlphaLISA for IL-10 secretion from human PBMCs by stimulation with the 5 selected microbiota proteins at doses of 0.5, 0.25, 0.1 and 0.025 µM (final concentration).

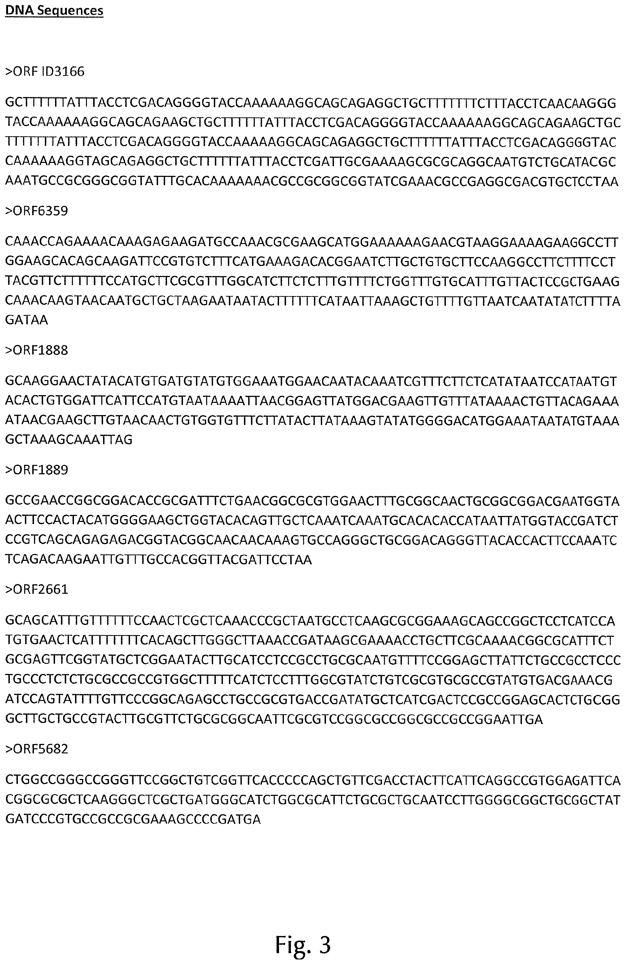

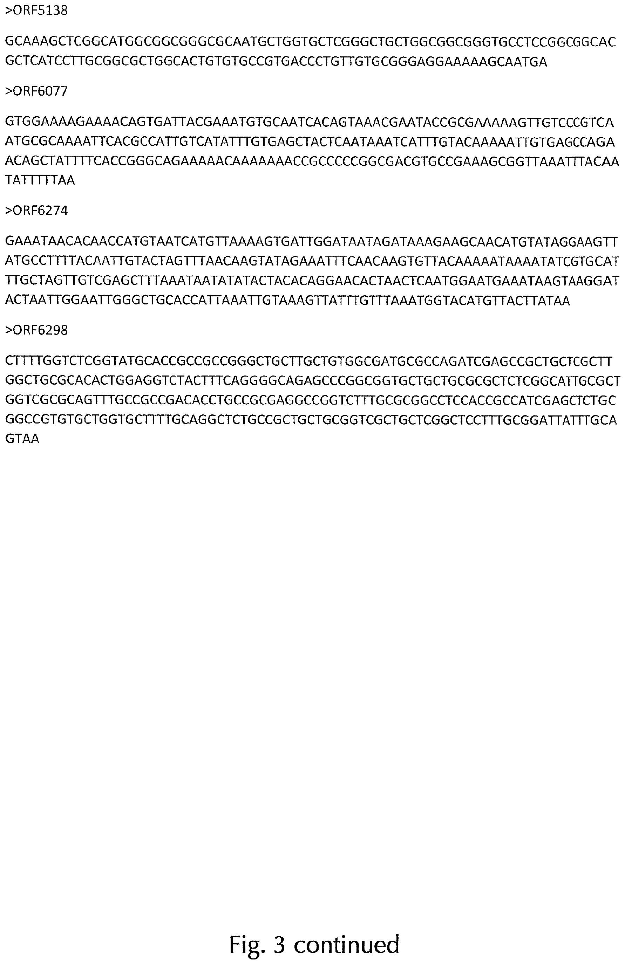

- Figure 3:: shows exemplified DNA sequences encoding the 10 exemplified proteins inducing IL-10, as identified in Example 1.

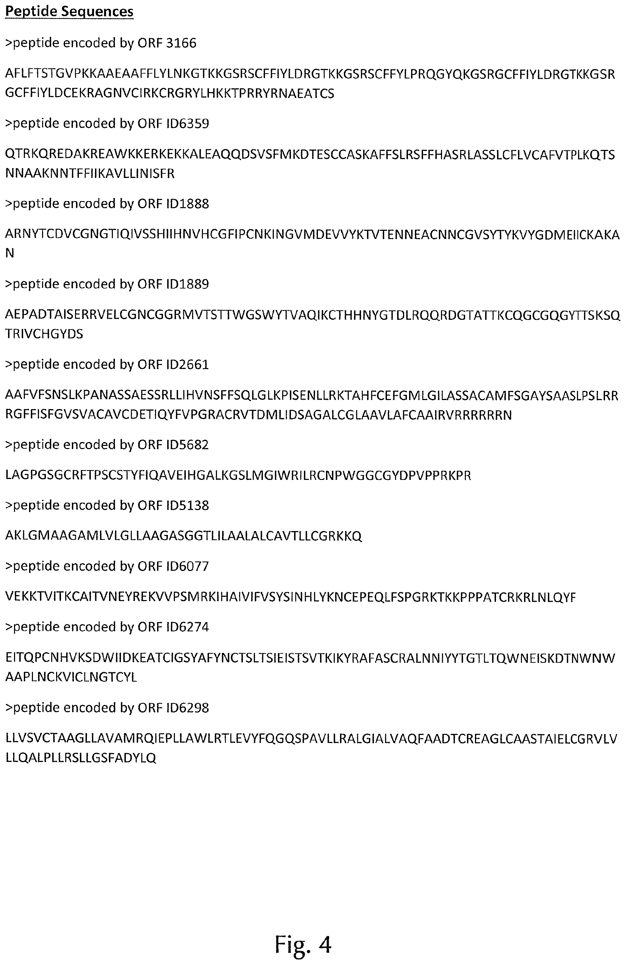

- Figure 4:: shows amino acid sequences of the 10 exemplified proteins inducing IL-10, as identified in Example 1.

### EXAMPLES

In the following, particular examples illustrating various embodiments and aspects of the invention are presented. However, the present invention shall not to be limited in scope by the specific embodiments described herein. The following preparations and examples are given to enable those skilled in the art to more clearly understand and to practice the present invention. The present invention, however, is not limited in scope by the exemplified embodiments, which are intended as illustrations of single aspects of the invention only, and methods which are functionally equivalent are within the scope of the invention. Indeed, various modifications of the invention in addition to those described herein will become readily apparent to those skilled in the art from the foregoing description, accompanying figures and the examples below. All such modifications fall within the scope of the appended claims.

### Example 1: Identification of human microbiota-derived proteins inducing IL-10 release from human cells

The aim of this study was to identify proteins expressed by human microbiota, which are able to induce secretion of IL-10 from human immune cells. To this end, a library of proteins expressed by human microbiota was screened to identify proteins inducing secretion of IL-10 from human immune cells.

### Experimental Procedures:

### Library: In silico method

A compound library of secreted proteins from gut commensal bacteria was generated by an *in silico*-based approach. The library included more than 12,000 proteins predicted from human gut microbiome catalogues and from bacterial species known for their role in immune modulation. To obtain the library, bacterial proteins having a length from 50 to 350 amino acids were screened for the presence of secretory signal peptides using the bioinformatic tool Phobius and were annotated using HMMSCAN and the PFAM database. A cut-off at 75% was applied to reduce sequence redundancy.

In view of the relevance of small cysteine-rich proteins in immune modulation an additional selection criterion was applied to identify cysteine-rich proteins: at least two cysteines were required to be present to form a disulphide bond. To ensure correct synthesis and folding *in vitro,* the amino acid sequences corresponding to the signal peptide were removed.

### Library: Cell free proteins synthesis and quantification

The protein library was generated using an *Escherichia coli* Cell Free kit suitable for generation of disulphide bonds (RTS 100 E. coli Disulfide Kit; Biotechrabbit, Hennigsdorf, Germany) according to the supplier's protocol. The Cell-Free system is based on the continuous exchange between the reaction compartment, containing components for transcription and translation, and the feeding chamber, containing amino acids and other energy components, through a semipermeable membrane.

Heterologous protein expression using the transcriptional machinery of *E. coli* was improved through a codon optimization algorithm (Twist Bioscience, San Francisco, USA) applied to all the selected sequences. All synthetized ORFs were subcloned into pIVEX 2.4 vector (Biotechrabbit, Hennigsdorf, Germany) specifically designed for high-yield Cell-Free expression of His-tagged proteins.

For the detection of His-tagged proteins, the 6His Check kit Gold using the HTRF® technology (Cisbio, Codolet, France) was used according to the supplier's protocol. Proteins, previously diluted at 1:20 in 1X PBS, were quantified in 384 well plates against a standard curve of 6xHis GFP at 0.1µg/mL (ThermoFisher, Waltham, USA) diluted in serial dilution in the lysate used for the Cell-Free synthesis (lysate was also diluted at 1:20 in 1X PBS).

### E. coli production of recombinant proteins

DNA from positive hits was subcloned in pET-28a vector carrying an N-terminal 6xHis-Tag (Twist Bioscience, San Francisco, USA) and then transformed in *E. coli* BL21(DE3) thermo competent cells (New England Biolabs, Ipswich, MA, USA). For the expression of recombinant proteins, pre-cultures of BL21(DE3) clones were performed in LB-medium at 30°C under shaking (180 rpm) conditions. Cultures were made under the same conditions and the induction was started when an OD600 of 0.4 - 0.8 was reached by using 0.5 mM IPTG. Depending on the properties of each protein, induction was performed for 2 hours to overnight.

Cultures were centrifuged for 15 min at 4°C, 4500 rpm. Supernatants were removed and the pellets were frozen at -80°C to break the cells. Pellets were then thawed and resuspended in 1X BugBuster® (Novagen®, Merck KGaA, Darmstadt, Germany) supplemented with Benzonase® Nuclease (Sigma-Aldrich, St. Louis, USA) and Lysozyme (Sigma-Aldrich, St. Louis, USA). Samples were incubated at room temperature by gentle shaking and centrifuged at 4°C, 15,000 g for 30 minutes. Soluble proteins were purified from supernatants onto Nickel packed columns (Protino®, Macherey-Nagel, Düren, Germany) according to the supplier's protocol. Proteins produced in inclusion bodies were solubilized from pellets using 8M urea. Imidazole (250 mM), used for proteins elution, was removed by buffer exchange using 3kDa cutoff filters (Amicon®, Merck Millipore Ltd., Burlington, USA). Proteins were visualized on 12% Bis-Tris acrylamide gels (ThermoFisher, Waltham, USA) stained with Coomassie Blue (Imperial protein Stain; ThermoFisher, Waltham, USA) and detected by Western Blot using the 6X-His Tag monoclonal antibody HIS.H8 (ThermoFisher, Waltham, USA) diluted at 1 :3000 and revealed using the secondary antibody Goat anti-Mouse IgG H+L WesternDot625 (ThermoFisher, Waltham, USA). Purified proteins were quantified by Bradford protein assay (Bradford M (1976) A rapid and sensitive method for the quantitation of microgram quantities of protein utilizing the principle of protein-dye binding. Anal. Biochem. 72:248-254).

### IL10 screening

IL-10 screening of the microbiota protein library was performed on CD14 depleted human peripheral blood mononuclear cells (PBMCs). This cellular model was chosen to reduce the background due to cell wall components and other bacterial contaminants possibly present in the lysate of the Cell Free synthesis kit on the synthesised (but not purified) proteins.

### CD14-depleted PBMCs:

PBMCs were isolated from buffy coats as follows: 80 ml PBS were added to 50 ml blood; 4 SepMate™-50 IVD tubes (Stemcell Technologies, Vancouver, Canada) were filled with 15 mL of Ficoll® (Ficoll® Paque Plus; Sigma-Aldrich, St. Louis, USA) per donor, then 30 ml of PBS-diluted blood were gently added. Samples were centrifuged for 20 min at 1200 g at room temperature and washed three times with PBS. To lyse the red blood cells, pellets were resuspended in Red Blood Cells Lysis buffer 1X (Miltenyi Biotec, Bergisch Gladbach, Germany) and incubated for 10 min at room temperature. Cells were then washed with MACS buffer and counted.

PBMC depletion was performed using the CD14 Microbeads kit (Miltenyi Biotec, Bergisch Gladbach, Germany) according to the supplier's protocol. Depleted monocytes were resuspended in Iscove's Modified Dulbecco's Medium (IMDM; GIBCO™, Life Technologies, Carlsbad, USA) supplemented with 1 % L-Glutamine (Sigma-Aldrich, St. Louis, USA), 1 % Penicillin-Streptomycin (Sigma-Aldrich, St. Louis, USA) and 10 % of heat-inactivated FBS (Sigma-Aldrich, St. Louis, USA).

### IL-10 screening:

Screening was performed in 384 well plates in a final volume of 60 µl. PBMCs were seeded at 72,000 cells/well by multidrop (Hamilton Robotics, Martinsried, Germany) and stimulated for 72 hours with 10% (vol/vol) of the library (proteins) previously diluted at 1:10 with PBS in a humidified 5% CO₂ atmosphere at 37°C. The *E. coli* lysate included in the cell free kit was used (at the same dilution as the library) as negative control. Phytohaemagglutinin (PHA) at 10 µg/ml (0.087 µM) was used as positive control. To ensure technical robustness, the screening was performed on CD14 depleted PBMCs from at least two different donors.

IL-10 secretion was measured by AlphaLISA® (IL10 (human) AlphaLISA Detection Kit; PerkinElmer, Waltham MA, USA) on the undiluted supernatants according to the supplier's protocol. Results were expressed as AlphaLISA signal (Counts). Results were considered as positive hits, either when at least a single raw data signal (of the two signals of the two PBMC donors) was higher than the corresponding plate mean + 3SD (Standard Deviation) or when both raw data signals (of the two signals of the two PBMC donors) were higher than the corresponding plate means + 2SD (Standard Deviation). In order to avoid false positives, the concentration of the potential hits obtained by the primary screening was compared to that of the corresponding plate mean.

Potential hits were then validated by a new round of Cell-Free synthesis and test on CD14 depleted PBMCs from several donors (as described above). Further characterization was performed on recombinant proteins produced in the *E. coli* BL21 (DE3) strain transformed with a pET-28a vector containing the target sequence (as described above, see paragraph "E. coli production of recombinant proteins"). Alternatively, a cell-free production of some proteins was performed by a commercial supplier (Synthelis, La Tronche, France).

### Results

### Screening

A total of 11904 proteins of the library was screened on CD14 depleted PBMCs in order to identify proteins able to stimulate IL-10 secretion from human PBMCs. From various potential hits obtained in the primary screening, so far ten proteins were confirmed in the second round of cell-free synthesis. These microbiota proteins, which are able to stimulate IL-10 secretion from human PBMCs, include ID3166 (SEQ ID NO: 1); ID6359 (SEQ ID NO: 2); ID1888 (SEQ ID NO: 3); ID1889 (SEQ ID NO: 4); ID2661 (SEQ ID NO: 5); ID5682 (SEQ ID NO: 6); ID5138 (SEQ ID NO: 7); ID6077 (SEQ ID NO: 8); ID6274 (SEQ ID NO: 9); and ID6298 (SEQ ID NO: 10).

Results of the AlphaLISA for IL-10 secretion from human PBMCs of the second round of cell-free synthesis are shown in Figure 1. The data show that ten IL-10 secretagogue proteins were identified by screening of a human microbiome metasecretome protein library on stimulation of IL-10 release from human PBMCs. These microbiota proteins induce IL-10 release from human immune cells.

### Example 2: Dose-response of selected IL-10 inducing microbiota proteins

Microbiota proteins ID3166 (SEQ ID NO: 1); ID2661 (SEQ ID NO: 5); ID5682 (SEQ ID NO: 6); and ID5138 (SEQ ID NO: 7) were selected. These proteins contain 121 (ID3166), 144 (ID2661), 58 (ID5682) and 44 (ID5138) amino acids, respectively, corresponding to a size of 14, 15.5, 6 and 4 kDa respectively.

Before testing, the proteins were purified. In view thereof, CD14 depletion of PBMCs was not required, since the background induced by the residual contaminants present in the purified proteins is very low. Therefore, the cellular assays of Example 2 to characterize the purified proteins were performed on total PBMCs.

The selected proteins, were tested at concentrations of 0.5, 0.25, 0.1 and 0.025 µM (final concentration) on PBMCs. Cells were seeded in 384 well plates at 72,000 cells/well in a final volume of 60 µl and stimulated with either the purified proteins (10 % vol/vol) or the relative controls (positive and negative) for 24 hours in a humidified 5% CO₂ atmosphere at 37°C. Samples were tested in duplicate on at least two different PBMCs donors. All the dilutions were made in PBS.

Secretion of IL-10 was measured by AlphaLISA and compared to those of PBS (negative control) and PHA at 10 µg/ml (positive control), essentially as described above.

Results of the AlphaLISA for IL-10 secretion from human PBMCs at different doses of the selected microbiota proteins are shown in Figure 2. The data show that all the tested proteins are as effective (ID5138) as PHA (positive control) or even more effective (ID 3166; ID2661; and ID5682) than PHA in inducing IL-10 secretion from PBMCs. Moreover, microbiota proteins ID3166 and ID5682 show higher effectivity than PHA even at the lowest doses tested.

**TABLE OF SEQUENCES AND SEQ ID NUMBERS (SEQUENCE LISTING):**

| **SEQ ID NO** | **Sequence** | **Remarks** |
|---|---|---|
| SEQ ID NO: 1 | AFLFTSTGVPKKAAEAAFFLYLNKGTKKGSRSCFFIY LDRGTKKGSRSCFFYLPRQGYQKGSRGCFFIYLDR GTKKGSRGCFFIYLDCEKRAGNVCIRKCRGRYLHK KTPRRYRNAEATCS | ID3166 |
| SEQ ID NO: 2 | QTRKQREDAKREAWKKERKEKKALEAQQDSVSF MKDTESCCASKAFFSLRSFFHASRLASSLCFLVCAFV TPLKQTSNNAAKNNTFFIIKAVLLINISFR | ID6359 |
| SEQ ID NO: 3 | ARNYTCDVCGNGTIQIVSSHIIHNVHCGFIPCNKI [?] NGVMDEVVYKTVTENNEACNNCGVSYTYKVYG DMEIICKAKAN | ID1888 |
| SEQ ID NO: 4 | AEPADTAISERRVELCGNCGGRMVTSTTWGSWYT[?] VAQIKCTHHNYGTDLRQQRDGTATTKCQGCGQ GYTTSKSQTRIVCHGYDS | ID1889 |
| SEQ ID NO: 5 | AAFVFSNSLKPANASSAESSRLLIHVNSFFSQLGLKP ISENLLRKTAHFCEFGMLGILASSACAMFSGAYSAA SLPSLRRRGFFISFGVSVACAVCDETIQYFVPGRAC RVTDMLIDSAGALCGLAAVLAFCAAIRVRRRRRRN | ID2661 |
| SEQ ID NO: 6 | LAGPGSGCRFTPSCSTYFIQAVEIHGALKGSLMGI WRILRCNPWGGCGYDPVPPRKPR | ID5682 |
| SEQ ID NO: 7 | AKLGMAAGAMLVLGLLAAGASGGTLILAALALCA I VTLLCGRKKQ | D5138 |
| SEQ ID NO: 8 | VEKKTVITKCAITVNEYREKVVPSMRKIHAIVIFVSYSI INHLYKNCEPEQLFSPGRKTKKPPPATCRKRLNLQ YF | D6077 |
| SEQ ID NO: 9 | EITQPCNHVKSDWIIDKEATCIGSYAFYNCTSLTSIE I ISTSVTKIKYRAFASCRALNNIYYTGTLTQWNEISK DTNWNWAAPLNCKVICLNGTCYL | D6274 |
| SEQ ID NO: 10 | LLVSVCTAAGLLAVAMRQIEPLLAWLRTLEVYFQG I QSPAVLLRALGIALVAQFAADTCREAGLCAASTAIE LCGRVLVLLQALPLLRSLLGSFADYLQ | D6298 |
| SEQ ID NO: 11 | GCTTTTTATTTACCTCGACAGGGGTACCAAAA [?] AAGGCAGCAGAGGCTGCTTTTTTCTTTACCTC AACAAGGGTACCAAAAAAGGCAGCAGAAGCT GCTTTTTATTTACCTCGACAGGGGTACCAAAA AAGGCAGCAGAAGCTGCTTTTTTATTTACCTC GACAGGGGTACCAAAAAGGCAGCAGAGGCTG CTTTTTTATTTACCTCGACAGGGGTACCAAAAA AGGTAGCAGAGGCTGCTTTTTTATTTACCTCGA TTGCGAAAAGCGCGCAGGCAATGTCTGCATAC GCAAATGCCGCGGGCGGTATTTGCACAAAAAA ACGCCGCGGCGGTATCGAAACGCCGAGGCGA CGTGCTCCTAA | D3166 |
| SEQ ID NO: 12 | CAAACCAGAAAACAAAGAGAAGATGCCAAAC GCGAAGCATGGAAAAAAGAACGTAAGGAAAA GAAGGCCTTGGAAGCACAGCAAGATTCCGTGT CTTTCATGAAAGACACGGAATCTTGCTGTGCTT CCAAGGCCTTCTTTTCCTTACGTTCTTTTTTCCAT GCTTCGCGTTTGGCATCTTCTCTTTGTTTTCTGG TTTGTGCATTTGTTACTCCGCTGAAGCAAACAA GTAACAATGCTGCTAAGAATAATACTTTTTTCAT AATTAAAGCTGTTTTGTTAATCAATATATCTTTTA GATAA | ID6359 |
| SEQ ID NO: 13 | GCAAGGAACTATACATGTGATGTATGTGGAAA TGGAACAATACAAATCGTTTCTTCTCATATAATC CATAATGTACACTGTGGATTCATTCCATGTAAT AAAATTAACGGAGTTATGGACGAAGTTGTTTAT AAAACTGTTACAGAAAATAACGAAGCTTGTAAC AACTGTGGTGTTTCTTATACTTATAAAGTATATG GGGACATGGAAATAATATGTAAAGCTAAAGCA AATTAG | ID1888 |
| SEQ ID NO: 14 | GCCGAACCGGCGGACACCGCGATTTCTGAAC GGCGCGTGGAACTTTGCGGCAACTGCGGCGG ACGAATGGTAACTTCCACTACATGGGGAAGCT GGTACACAGTTGCTCAAATCAAATGCACACAC CATAATTATGGTACCGATCTCCGTCAGCAGAG AGACGGTACGGCAACAACAAAGTGCCAGGGC TGCGGACAGGGTTACACCACTTCCAAATCTCA GACAAGAATTGTTTGCCACGGTTACGATTCCTA A | ID1889 |
| SEQ ID NO: 15 | GCAGCATTTGTTTTTCCAACTCGCTCAAACCC GCTAATGCCTCAAGCGCGGAAAGCAGCCGGC TCCTCATCCATGTGAACTCATTTTTTCACAGCT TGGGCTTAAACCGATAAGCGAAAACCTGCTTC GCAAAACGGCGCATTTCTGCGAGTTCGGTATG CTCGGAATACTTGCATCCTCCGCCTGCGCAAT GTTTTCCGGAGCTTATTCTGCCGCCTCCCTGCC CTCTCTGCGCCGCCGTGGCTTTTTCATCTCCTTT GGCGTATCTGTCGCGTGCGCCGTATGTGACGA AACGATCCAGTATTTTGTTCCCGGCAGAGCCT GCCGCGTGACCGATATGCTCATCGACTCCGCC GGAGCACTCTGCGGGCTTGCTGCCGTACTTGC GTTCTGCGCGGCAATTCGCGTCCGGCGCCGG CGCCGCCGGAATTGA | ID2661 |
| SEQ ID NO: 16 | CTGGCCGGGCCGGGTTCCGGCTGTCGGTTCA CCCCCAGCTGTTCGACCTACTTCATTCAGGCCG TGGAGATTCACGGCGCGCTCAAGGGCTCGCT GATGGGCATCTGGCGCATTCTGCGCTGCAATC CTTGGGGCGGCTGCGGCTATGATCCCGTGCC GCCGCGAAAGCCCCGATGA | ID5682 |
| SEQ ID NO: 17 | GCAAAGCTCGGCATGGCGGCGGGCGCAATGC TGGTGCTCGGGCTGCTGGCGGCGGTGCCTC CGGCGGCACGCTCATCCTTGCGGCGCTGGCA CTGTGTGCCGTGACCCTGTTGTGCGGGAGGA AAAAGCAATGA | ID5138 |
| SEQ ID NO: 18 | GTGGAAAAGAAAACAGTGATTACGAAATGTGC AATCACAGTAAACGAATACCGCGAAAAAGTTG TCCCGTCAATGCGCAAAATTCACGCCATTGTCA TATTTGTGAGCTACTCAATAAATCATTTGTACAA AAATTGTGAGCCAGAACAGCTATTTTCACCGG GCAGAAAAACAAAAAAACCGCCCCCGGCGAC GTGCCGAAAGCGGTTAAATTTACAATATTTTTA A | ID6077 |
| SEQ ID NO: 19 | GAAATAACACAACCATGTAATCATGTTAAAAGT GATTGGATAATAGATAAAGAAGCAACATGTAT AGGAAGTTATGCCTTTTACAATTGTACTAGTTTA ACAAGTATAGAAATTTCAACAAGTGTTACAAAA ATAAAATATCGTGCATTTGCTAGTTGTCGAGCT TTAAATAATATATACTACACAGGAACACTAACT CAATGGAATGAAATAAGTAAGGATACTAATTG GAATTGGGCTGCACCATTAAATTGTAAAGTTAT TTGTTTAAATGGTACATGTTACTTATAA | ID6274 |
| SEQ ID NO: 20 | CTTTTGGTCTCGGTATGCACCGCCGCCGGGCT GCTTGCTGTGGCGATGCGCCAGATCGAGCCG CTGCTCGCTTGGCTGCGCACACTGGAGGTCTA CTTTCAGGGGCAGAGCCCGGCGGTGCTGCTG CGCGCTCTCGGCATTGCGCTGGTCGCGCAGTT TGCCGCCGACACCTGCCGCGAGGCCGGTCTTT GCGCGGCCTCCACCGCCATCGAGCTCTGCGG CCGTGTGCTGGTGCTTTTGCAGGCTCTGCCGC TGCTGCGGTCGCTGCTCGGCTCCTTTGCGGAT TATTTGCAGTAA | ID6298 |

## Claims

1. An isolated protein of non-pathogenic microbiota, or a fragment or sequence variant thereof, which is capable of inducing and/or enhancing IL-10 secretion from human cells.

2. The protein, or a fragment or sequence variant thereof, according to claim 1, wherein the protein is a protein of the metasecretome of non-pathogenic microbiota.

3. The protein, or a fragment or sequence variant thereof, according to claim 1 or 2, wherein the protein is a protein of human non-pathogenic microbiota.

4. The protein, or a fragment or sequence variant thereof, according to any one of claims 1 to 3, wherein the protein is a bacterial protein, preferably of the human gastrointestinal tract microbiota metasecretome.

5. The protein, or a fragment or sequence variant thereof, according to any one of claims 1 to 4, wherein the protein comprises at least two cysteine residues.

6. The protein, or a fragment or sequence variant thereof, according to any one of claims 1 to 5, wherein the protein has a length of no more than 400 amino acids.

7. The protein, or a fragment or sequence variant thereof, according to any one of claims 1 to 6, wherein the human cells are human immune cells, preferably PBMCs.

8. The protein, or a fragment or sequence variant thereof, according to any one of claims 1 to 7, wherein IL-10 secretion from human cells stimulated with said protein, or the fragment or sequence variant thereof, is higher than IL-10 secretion from human cells stimulated with an *E. coli* lysate.

9. The protein, or a fragment or sequence variant thereof, according to any one of claims 1 to 8, wherein the protein, or the fragment or sequence variant thereof, comprises or consists of an amino acid sequence sharing at least 80% sequence identity with any one of SEQ ID NOs 1 to 10.

10. The protein according to any one of claims 1 to 9, wherein the protein has an amino acid sequence according to SEQ ID NO: 1.

11. A nucleic acid comprising a polynucleotide encoding the protein, or the fragment or sequence variant thereof, according to any one of claims 1 to 10.

12. An expression cassette comprising a polynucleotide encoding the protein, or the fragment or sequence variant thereof, according to any one of claims 1 to 10 and, operably linked thereto, a regulatory element, preferably for expression in a prokaryotic cell, such as a bacterium.

13. A genetically engineered bacterium capable of inducing and/or enhancing IL-10 secretion from a human cell, the bacterium comprising the nucleic acid according to claim 11 or the expression cassette according to claim 12.

14. A pharmaceutical composition comprising the protein, or the fragment or sequence variant thereof, according to any one of claims 1 to 10, the nucleic acid according to claim 11 or the bacterium according to claim 13 and, optionally, a pharmaceutically acceptable excipient or carrier.

15. The protein, or the fragment or sequence variant thereof, according to any one of claims 1 to 10, the nucleic acid according to claim 11, the bacterium according to claim 13, or the pharmaceutical composition according to claim 14 for use in medicine.
